# EUROPEAN PATENT SPECIFICATION

(11) **EP 0 500 699 B1**
(45) Date of publication and mention of the grant of the patent: **10.06.1998**
(21) Application number: 90917076.3
(22) Date of filing: 02.11.1990
(51) Int. Cl.: A61K 39/112

(54) **CROSS-PROTECTIVE SALMONELLA VACCINES**
KREUZSCHÜTZENDE SALMONELLA IMPFSTOFFE
VACCINS DE PROTECTION CROISEE CONTRE LA SALMONELLA

(30) Priority: 03.11.1989 US 431597
(43) Date of publication of application: 02.09.1992
(73) Proprietor: WASHINGTON UNIVERSITY, St. Louis, MO 63130 (US)
(72) Inventor: CURTISS, Roy, III, St. Louis, MI 63112 (US); MUNSON, Maryann, St. Louis, MI 63112 (US)
(74) Representative: Goldin, Douglas Michael
(86) International application number: US9006503
(87) International publication number: WO9106317

(56) References cited:
- EP-A- 0 249 449
- WO-A-90/11687
- Infection and Immunity, Vol. 55(12) issued December 1987, CURTISS, et al., Salmonella typhimurium Deletion Mutants Lacking Adenylate Cyclase and Cyclic AMP Receptor Protein are Avirulent and Immunogenic, pages 3035-3043, see entire document, particularly the Discussion.
- FEMS Microbiology Letters, Vol. 28, issued 1985, STEVENSON et al., "Galactose epimeraseless (Ga1E) mutant G30 of Salmonella typhimurium is a good potential live oral vaccine carrier for fimbrial antigens", pages 317-321, see entire document.
- J. Infection Disease, Vol. 156(1), issued July 1987, HONE, et al., "Construction of Defined ga1E Mutants of Salmonella for Use as Vaccines", pages 167-174, see entire document.
- Infection and Immunity, Vol. 34(3), issued December 1981, FORMAL et al, "Construction of a Potential Divalent Vaccine Strain: Introduction of Shigella sonnei Form I Antigen genes into the ga1E Salmonella typhi Tyzla Typhoid Vaccine Strain", pages 746-750, see Discussion.
- Vaccine, Vol. 6, issued April 1988, CURTISS et al., "Avirulent Salmonella typhimurium D cya Dcrp Oral vaccine strains expressing a streptococcal colonization and virulence antigen, pages 155-160, see entire document.
- Infection and Immunity, Vol. 55(4), issued April 1987, NNALUE et al., "Tests of the Virulence and Live Vaccine Efficacy of Auxotrophic and ga1E Derivatives of Salmonella Choleraesuis" pages 955-962, see Introduction an Discussion.
- Escherichia coli and Salmonella Typhimurium, chapters 80-81
- The Journal of Infectious Diseases, vol.150 (3), 1984, pages 436-449
- Infection and Immunity, 1988, vol.56(5), pages 1326-1333

## Description

### Technical Field

This invention pertains to materials and methodologies for immunizing individuals to protect against infections by gram-negative bacteria, and more particularly to vaccine compositions which are comprised of avirulent Salmonella, and which are able to induce immunity not only to homologous and heterologous Salmonella species, but also to induce cross-protective immunity to other gram-negative enterobacteria.

### References Cited in the Application

Arnow (1937), J. Biol. Chem. 118:531.
Chart and Griffiths (1985), J. Gen. Microbiol. 131:1503.
Curtiss and Kelly (1987), Infect. Imm. 55:3035.
Ernst et al. (1978), J. Bacteriol. 135:928.
Finlay et al (1989), Science 243:1059.
Galan and Curtiss III (1989), Proc. Natl. Acad. Sci. USA 86:6383.
Galan and Curtiss III (1989), Microbial Pathogen. 6:433.
Germanier and Furer (1971), Infect. Immun. 4:663.
Gulig and Curtiss III (1987), Infect. Immun. 55:2891.
Liefson (1936), Am. J. Hyg. 24:423.
Miller et al. (1989), Proc. Natl. Acad. Sci. USA 86:5054.
Mullis and Faloona (1987), Methods Enzymol. 155:335.
Tanomoto and Homma (1982), J. Biochem. 91:741.
Tsai and Frasch (1983), Anal. Biochem. 119:115.

### Background of the Invention

There are more than 1800 serotypes of Salmonella combined into five major and many minor antigenic groups as defined by the O and H antigens. Nevertheless, many consider that only three species of Salmonella exist: S. typhi, S. choleraesuis, and S. enteritidis, the last of which contains the vast majority of serotypes. Most of the S. enteritidis serotypes (which herein are listed as species) have a relatively low host specificity and thus can infect a diversity of animal species including humans. Salmonella infection in humans affects predominantly the very young, the elderly and immune-compromised individuals, and in most cases is caused by contaminated food poultry products. It is also relevant to note that an expert committee on salmonellosis from the WHO Surveillance Program for the Control of Foodborne Infections and Intoxications concluded in 1987 that human salmonellosis is a global problem representing 60 to 80 percent of all reported cases of foodborne disease. The seven most prevalent Salmonella species currently isolated from poultry and associated with human disease are S. enteritidis, S. typhimurium, S. heidelberg, S. infantis, S. agona, S. saint-paul, and S. montevideo. Most of these Salmonella species cause gastroenteritis in humans, with possible persistence and continued shedding. It is estimated, however, that in the U.S. less than one percent of these Salmonella infections are reported and accurately diagnosed. Most Salmonella are transmitted through the food chain by fecal contamination of carcasses during the dressing operation. Studies conducted to investigate the magnitude of this problem have found that from one to fifty percent of the poultry carcasses are contaminated. A more recent concern is associated with the transmission of S. enteritidis through the egg directly to the consumer, presumably because some strains of S. enteritidis can persistently infect the ovaries of laying hens. This has become increasingly prevalent in the northeastern and mid-Atlantic states and has led not only to numerous infections but some deaths. It is therefore evident that transmission of Salmonella to humans through persistent infection of farm animals and contamination of meat and eggs constitutes an important public health problem. An additional complication is the increasing isolation of drug-resistant Salmonella which account for 20 to 25 percent of the human cases. It is believed that subtherapeutic amounts of antibiotics in animal feed select for resistant bacterial which eventually infect humans, thus exacerbating the public health problem.

E. coli infection of the respiratory tract in poultry to cause airsacculitis, pneumonia and septicemia accounts for major losses in the poultry industry. The majority of all E. coli-induced colisepticiemia in poultry are caused by E. coli strains having one of three O antigens: O1, O2 and O78. Colisepticemia is thought to occur via inhalation of feces-contaminated dust. The precise location of E. coli deposition within the respiratory tract to cause disease is unknown. In addition to their ability to colonize the respiratory tract of birds, E. coli strains capable of causing colisepticemia have a set of virulence attributes that are very similar to those expressed by E. coli strains causing extraintestinal infections in humans and other animals. Thus, colicin V (ColV) plasmids have been found to be present in a significant proportion of strains causing colisepticemia in chickens and in strains causing meningitis in humans. The transfer of ColV plasmids into E. coli decreases its LD₅₀ when injected IP into mice and IV into chicks, and curing ColV plasmids from pathogenic strains decreases their virulence. The synthesis of Colicin V has not been implicated in this increase in lethality. This correlation between ColV plasmid presence and lethality prompted further characterization of ColV plasmids. ColV I-K94, a plasmid present in E. coli K94, a strain isolated from the feces of patients infected with Salmonella paratyphi B, has been found to contain the gene iss which blocks the action of, but not the formation of, the terminal complex of complement. The presence and expression of aerobactin genes, an efficient iron chelation system that is thought to play a role in virulence, has been shown to be highly correlated with the virulence of avian pathogenic E. coli strains, as well as with human clinical isolates from septicemia, pyelonephritis, and lower urinary tract infection. The aerobactin genes have been shown to reside on ColV plasmids in both avian and human pathogenic isolates. Another attribute that has been shown to play a role in the virulence of human extraintestinal E. coli isolates is the capsular antigen K1. Approximately 80% of E. coli causing newborn meningitis express K1, and a significant proportion of E. coli causing urinary tract infections also have K1. The K1 antigen is thought to shield the cell from activating complement. The role of K1 as a virulence factor in avian strains has not been studied, but a significant proportion of strains causing colisepticemia are O1:K1 and O2:K1. The K80 capsule found with O78 strains may have virulence-enhancing properties similar to K1.

Pili have been shown to play an important role in the adherence of E. coli to host tissue and to subsequent pathogenesis. This also seems to be true for the E. coli causing colisepticemia in that vaccines composed of purified pili are able to protect chickens against subsequent challenge. In addition to similar virulence factors between E. coli strains causing colisepticemia in poultry and extraintestinal infections in humans, there is also overlap in the serotypes associated with these diseases. As cited above, avian colisepticemia is usually caused by strains of O serotype O1, O2 and O78. The K1 antigen is usually associated with O1 and O2 strains, and thus O1:K1 and O2:K1 serotypes account for a large proportion of avian colisepticemia. These serotypes are also associated with human diseases such as newborn meningitis and urinary tract infection. Clonal analysis of O2:K1 strains isolated from newborn meningitis, urinary tract infection and avian colisepticemia show these strains to be very similar in terms of outer membrane protein profiles and electrophoretic mobility of enzymes. In addition, these strains are closely related to the O1:K1 strains isolated from urinary tract infection, septicemia and the newborn meningitis.

It is evident, therefore, that in addition to the extensive morbidity and mortality due to colisepticemia which has adverse economic consequences for the poultry industry, it is possible that poultry might constitute a reservoir for transmission through the food chain of E. coli strains capable of causing extraintestinal infections in humans. If so, prevention of these infections in poultry would eliminate this putative, but as yet unproven, human public health problem.

Infection and colonization by Salmonella is a consequence of oral ingestion of Salmonella-contaminated materials. A wild-type lipopolysaccharide (LPS) with repeating O-side chains seems to be essential for initial colonization since rough strains lacking O-side chains or part of the core fail to penetrate through the mucin and glycocalyx covering the intestinal wall and pass right through the intestinal tract. Although it is quite possible that pili, flagella and various mannose-resistant adhesins may permit Salmonella to attach to cells lining the intestinal wall, the absence of any one of these by mutation seems to be without effect on intestinal colonization and causation of disease. In S. typhimurium, invasion of cells in the intestinal mucosa is dependent upon the activities of four genes, three of which constitute an operon and which specify the invasion mechanism. S. typhimurium Inv⁻ mutants, in addition to having a 100-fold reduced ability to invade cells in culture, invade cells of the intestinal mucous less well and have an LD₅₀ 60 to 100 times higher than wild-type strains by the oral route of infection. The inv genes are not necessary for infection by other routes, however, since the LD₅₀ for wild-type and Inv⁻ mutants are the same for intraperitoneal inoculation. It is known that S. typhimurium initially attaches to, invades and persists in the gut-associated lymphoid tissue (GALT or Peyer's patches) prior to reaching deeper tissues such as the mesenteric lymph nodes, liver and spleen. Effective colonization of these deeper tissues is dependent upon the presence of a virulence plasmid in certain invasive Salmonella species as well as on a number of chromosomal genes. Only the serotypes S. typhimurium, S. enteritidis, S. dublin, S. choleraesuis, S. gallinarum and S. pullorum are frequently endowed with a virulence plasmid that governs virulence when infection is initiated by the oral route. Several studies have indicated that strains lacking this virulence plasmid are still able to colonize the intestinal tract but are less able to reach and/or colonize the liver and spleen. Specifically a 28 kDa plasmid-encoded protein is largely responsible for this phenotype in S. typhimurium. The DNA sequence encoding this 28 kDa plasmid-encoded protein hybridizes with the same sequence in the virulence plasmids from all of the above invasive Salmonella species. Therefore, the 28 kDa protein may play the same role in all these invasive species.

Continued production of LPS in vivo is also essential for the ability of Salmonella to cause invasive disease since absence of LPS renders Salmonella susceptible to nonspecific host defense mechanisms. Several genes which are involved in global regulation of other genes are necessary for Salmonella to efficiently colonize deep tissues and cause disease. Thus, Curtiss and Kelly demonstrated that S. typhimurium strains unable to synthesize adenylate cyclase and the cyclic AMP receptor were avirulent and immunogenic . More recently, S. typhimurium strains with mutations in the phoP gene, which regulates genes that allow Salmonella to survive in macrophages are totally avirulent but also immunogenic. Strains possessing mutations in phoQ (Miller et al. (1989) have the same phenotype as mutations in phoP. Hereinafter strains with mutations in either phoP or phoQ are referred to collectively as phoP mutants.

The known attributes associated with E. coli strains capable of causing colibacillosis are enumerated above. It is evident, however, that these E. coli which cause airsacculitis must presumably, like Salmonella, have a mechanism to invade through the membranes of the airsac and/or the lung since pericarditis can occur in the absence of septicemia. It is likely that outer membrane proteins are involved in this invasion as they are in invasion by Salmonella and Yersinia. As stated above, an essential attribute of E. coli strains causing septicemia is a very efficient means for iron sequestering. Low concentrations of approximately 10⁻¹⁸M iron found in serum are well below the concentrations needed for bacterial growth. Iron deprivations has been shown to induce changes in the cell's metabolism by synthesis of low molecular weight iron chelators and outer membrane proteins. It is these outer membrane proteins that are receptors for ferric-siderophore complexes and form part of the high-affinity iron acquisition system of E. coli. In this regard, it is relevant to note that antibodies against one of these iron-regulated outer membrane proteins (OMP) can protect turkeys against colibacillosis. Also, it should be noted that sera against an E. coli ferric enterochelin receptor cross-reacted with enterochelin receptor and several high-molecular-weight proteins produced by iron-stressed S. typhi cells.

In mice and humans (and presumably in other vertebrates) a common mucosal immune network exists such that presentation of antigens to the GALT triggers proliferation and dissemination of committed B-cells to all secretory tissues and glands in the body, with the ultimate production of secretory IgA (sIgA). sIgA, directed against specific surface antigens of pathogens that colonize on and/or pass through a mucosal surface, serves to block their colonization and invasion. There is also evidence to suggest that antigen-specific sIgA might facilitate antibody-dependent cytotoxicity mediated by phagocytic cells on the gut epithelium and lamina propria. Although a secretory immune response is inadequate to completely block infection by invasive pathogens, it does increase the dose of microorganisms necessary to cause disease. Consequently, its induction should decrease the likelihood of infection and contagious spread of pathogens such as Salmonella.

Avirulent mutants of S. typhimurium have been isolated which have the ability to stimulate protective immunity against infection with virulent Salmonella. Subsequently, it has been learned that many of these avirulent mutants retain the ability to attach to, invade and persist in the GALT . It has been reported that Salmonella mutants unable to synthesize adenylate cyclase and lacking the cyclic AMP receptor protein due to deletion (delta) mutants in the cya and crp genes, respectively, are completely avirulent and highly immunogenic when used for oral immunization of mice. It has also been learned that delta-cya and delta-crp S. typhimurium strains are avirulent for pigs and sheep and delta-cya delta-crp S. choeraesuis are avirulent and immunogenic for mice.

Mice immunized with S. typhimurium will frequently exhibit nonspecific resistance against challenge with heterologous bacterial strains for a month or so post-immunization, after which immunity becomes specific for S. typhimurium or other species with the same group antigen. In the nonspecific phase of the response, LPS seems to be responsible for stimulating production of activated macrophages although this might not be true in chickens which are at least insensitive to LPS toxicity. Although the immune response to T-independent O-antigen determinants is relatively specific for individual Salmonella groups, it is likely that there would be more immunological cross-reactivity associated with surface proteins in different O-antigen groups or to the lipid A-LPS core antigen which is common to all Salmonella. In this regard, little attention has been paid to the possibility of inducing cross-protective mucosal and humoral immunity by heterologous Salmonella caused by the presence of shared surface protein antigens and LPS core epitopes.

Many studies characterizing the avian immune system were reported more than 10 years ago. Chickens have a GALT and BALT, and the Harder gland, which is located ventrally and posteriomedially to the eyeball, contains antibody-secreting cells, and may play an important role in producing secretory antibodies for the upper respiratory tract. It is unknown whether the antibody secreting cells in this gland arise from antigen stimulation of the GALT or the BALT or of the Harder gland itself. Information about the existence of a common mucosal immune system in birds is generally lacking.
European Patent Application No. 0249449 discloses a avirulent strain of an enterobacterium containing a *gal* operon and eluding at least one defined non-revertible mutation in the *galE* gene and at least one further defined non-revertible attenuating mutation and a fragment of DNA containing a gene of interest. This avirulent strain may be provided as a vaccine composition.

### Disclosure of the Invention

The invention relates to live avirulent *Salmonella* and their use as vaccines for use in immunizing individuals to induce cross-protective immunity to reduce infection and colonization by homologous and heterologous *Salmonella* serotypes and by other gram-negative enteric bacteria.

Accordingly, one aspect of the invention is a vaccine for treatment of an individual for infections by gram-negative bacteria comprised of live avirulent *Salmonella* which are able to induce immunity to homologous and heterologous *Salmonella* serotypes and to other gram-negative enteric bacteria, wherein the *Salmonella* possess a mutation in a gene which encodes adenylate cyclase (*cya*) and/or cyclic AMP receptor protein (*crp*), and also possess a mutation in a gene encoding an enzyme in lipopolysaccharide synthesis which results in a reversibly rough phenotype, the amount of said live cells being sufficient to improve the resistance of the individual to infection by the gram-negative enteric bacteria, the *Salmonella* cells being present in a pharmaceutically acceptable carrier.

Another aspect of the invention is a vaccine for treatment of an individual for infections by gram-negative bacteria comprised of live avirulent *Salmonella* which are able to induce immunity to homologous and heterologous *Salmonella* serotypes and to other gram-negative enteric bacteria, wherein the *Salmonella* possess at least one mutation in a gene involved in the global regulation of pathogenicity of the *Salmonella*, and also possess a mutation in a gene regulating the synthesis of iron-regulated outer membrane proteins (OMP), such that the mutation leads to constitutive expression of iron regulated OMP, the amount of said live cells being sufficient to improve the resistance of the individual to infection by the gram negative enteric bacteria, the *Salmonella* cells being present in a pharmaceutically acceptable carrier.

Yet another aspect of the invention is a isolated avirulent *Salmonella* strain which is able to induce immunity to homologous and heterologous *Salmonella* serotypes and to other gram-negative enteric bacteria wherein the strain possesses a mutation in a gene which encodes adenylate cyclase (*cya*) and/or cyclic AMP receptor protein (*crp*), and also possesses a mutation in a gene encoding a enzyme in lipopolysaccharide synthesis which results in a reversibly rough phenotype.

The invention further provides use of live avirulent *Salmonella*, which possess at least one mutation in a gene involved in the global regulation of pathogenicity of the *Salmonella* and also possess a mutation in a gene encoding an enzyme in lipopolysaccharide synthesis which results in a reversibly rough phenotype, for the manufacture of a medicament for use in inducing immunity to heterologous *Salmonella* serotypes or to gram-negative bacteria other than *Salmonella*.

Still another aspect of the invention is a isolated avirulent *Salmonella* strain which is able to induce immunity to homologous and heterologous *Salmonella* serotypes and to other gram-negative enteric bacteria, wherein the strain possesses at least one mutation in a gene involved in the global regulation of pathogenicity of the *Salmonella*, and also possesses a mutation in a gene regulating the synthesis of iron-regulated outer membrane proteins (OMP), such that the mutation leads to constitutive expression of iron-regulated OMP.

### Brief Description of the Drawings

Figure 1 is a schematic diagram showing the structure of the lipopolysaccharide (LPS) of *S. typhimurium*.

Figure 2 is a photocopy of a Western immunoblot of whole bacterial extracts probed with serum from a rat immunized with viable Chi3306 cells. The bacterial extracts are from a variety of strains of E. coli and of Salmonella.

Figure 3 is a photocopy of a Western immunoblot of whole bacterial extracts probed with serum from birds immunized with viable Chi3985 cells, and from unimmunized control birds; the extracts are of E. coli Chi7122, and of S. typhimurium Chi3985, both strains grown in broth.

Figure 4 is a photocopy of a Western immunoblot of whole bacterial extracts probed with serum from a rabbit immunized with E. coli strain Chi7122 grown in broth. The bacterial extracts are of S. typhimurium and of E. coli grown in the presence of low and of high iron.

Figure 5 is a photocopy of a Western immunoblot of whole bacterial extracts probed with serum from a rabbit immunized with an outer membrane protein preparation of E. coli strain Chi7122. The bacterial extracts are of wild type and fur mutants of S. typhimurium grown in the presence of low and of high concentrations of iron.

### Modes for Carrying Out the Invention

### A. Definitions

"Recombinant host cells", "host cells", "cells" and other such terms denoting microorganisms are used interchangeably, and refer to cells which can be, or have been, used as recipients for recombinant vectors or other transferred DNA, and include the progeny of the original cell transfected. It is understood that the progeny of a single parental cell may not necessarily be completely identical in genomic or total DNA complement as the original parent, due to accidental or deliberate mutation. Progeny of the parental cell which are sufficiently similar to the parent to be characterized by a relevant property, for example, a phenotype conferred by the mutations described herein, e.g., the inability of delta-cya or delta-crp Salmonella mutants to ferment or to grown on the carbohydrates, maltose, mannitol, sorbitol, melibiose, citrate and glycerol is revealed by plating on a suitable fermentation indicator medium such as MacConkey Agar supplemented with 1 percent of the appropriate carbohydrate or by inability to grow on a mineral salts minimal medium supplemented with 0.5 percent of the carbohydrate, respectively, in contrast to all wild-type Salmonella which are able to ferment and/or use all of these carbohydrates.

"Gram negative bacteria" include cocci, nonenteric rods, enteric rods, and spirilla. The genera of gram negative bacteria include, for example, Neisseria, Spirillum, Pasteurella, Brucella, Yersinia, Francisella, Haemophilus, Bordetella, Escherichia, Salmonella, Shigella, Klebsiella, Proteus, Vibrio, Pseudomonas, Bacteroides, Acetobacter, Aerobacter, Agribacterium, Azotobacter, Spirilla, Serratia, Vibrio, Rhizobium, Chlamydia, Rickettsia, Trepanema, Spirillum and Fusobacterium,

"Gram positive bacteria" include cocci, nonsporulating rods, and sporulating rods. The genera of gram positive bacteria include, for example, Actinomyces, Bacillus, Clostridium, Corynebacterium, Erysipelothrix, Lactobacillus, Listeria, Mycobacterium, Myxococcus, Nocardia, Staphylococcus, Streptococcus, and Streptomyces.

"Enterobacteriaceae", also known collectively as enterics, include Escherichieae, Edwardsielleae, Salmonellae, Yersinia, Providencia, Serratia, Erwiniae, Citrobacterae, Enterobacterae, Shigellae, Klebsiellae, and Proteae.

"Mycobacteria" are defined on the basis of their distinctive staining property, i.e., they resist decolorization with acidified organic solvents, and on the presence of long chain (approximately 60 carbons) mycolic acids.

The gene symbols for mutant strains utilized herein are those described by Bachmann (1987), and Sanderson and Roth (1987). The symbols used for transposons, particularly Tn10, follow the convention described in Bukhari et al (1977).

As used herein, the term "reversibly rough phenotype" means that the strain grows a complete lipopolysaccharide when a carbohydrate for which it is auxotrophic is supplied, and produces an incomplete lipopolysaccharide coat when the carbohydrate is limiting or absent, such that the lipopolysaccharide core is exposed. Methods of detecting "rough" coats are known in the art, and examples of detecting mutants with reversibly rough phenotypes are disclosed infra.

As used herein, the term "constitutive expression" refers to the expression of a polypeptide under conditions when that expression is normally repressed. Methods of detecting constitutive expression are known in the art, and examples of some methods are provided infra.

An "individual" treated with a vaccine of the invention is defined herein as including all vertebrates, for example, mammals, including domestic animals and humans, various species of birds, including domestic birds, particularly those of agricultural importance. In addition, mollusks and certain other invertebrates have a primitive immune system, and are included as an "individual".

"Treatment" refers to the administration of the vaccine to an individual which yields a protective immune response, and includes prophylaxis and/or therapy.

"Transformation", as used herein, refers to the insertion of an exogenous polynucleotide into a host cell, irrespective of the method used for the insertion, for example, direct uptake, transduction, or conjugation. The exogenous polynucleotide may be maintained as a plasmid, or alternatively, may be integrated within the host genome.

As used herein, a "pathogenic microorganism" causes symptoms of a disease usually associated with the pathogen.

An "avirulent microorganism" is one which has the ability to colonize and replicate in an infected individual, but which does not cause disease symptoms usually associated with virulent strains of the same species of microorganism. Avirulent does not mean that a microbe of that genus or species cannot ever function as a pathogen, but that the particular microbe being used is avirulent with respect to the particular individual being treated. The microbes may belong to a genus or even a species that is normally pathogenic but must belong to a strain that is avirulent. Avirulent strains are incapable of inducing a full suite of symptoms of the disease that is normally associated with its virulent pathogenic counterpart. Avirulent strains of microorganisms may be derived from virulent strains by mutation.

The term "microbe" as used herein includes bacteria, protozoa, and unicellular fungi.

An "antigen" refers to a molecule containing one or more epitopes that will stimulate a host's immune system to make a secretory, humoral and/or cellular antigen-specific response. The term is also used interchangeably with "immunogen".

A "hapten" is a molecule containing one or more epitopes that does not itself stimulate a host's immune system to make a secretory, humoral or cellular response.

The term "epitope" refers to a site on an antigen or hapten to which an antibody or cell receptor specific to that site binds. An epitope could comprise 3 amino acids in a spatial conformation which is unique to the epitope; generally, an epitope consists of at least 5 such amino acids, and more usually, consists of at least 8-10 such amino acids. the term is also used interchangeably with "antigenic determinant" or "antigenic determinant site."

An "immunological response" to a composition or vaccine comprised of an antigen is the development in the host of a cellular and/or antibody-mediated immune response to the composition or vaccine of interest. Usually, such a response consists of the subject producing antibodies, B cells, helper T cells, suppressor T cells, and/or cytotoxic T cells directed specifically to an antigen or antigens included in the composition or vaccine of interest.

By "vaccine composition" or "vaccine" is meant an agent used to stimulate the immune system of an individual so that current harm is alleviated, or protection against future harm is provided.

"Immunization" refers to the process of inducing a continuing high level of antibody and/or cellular immune response which is directed against an antigen to which the organism has been previously exposed.

### B. General Description

The practice of the present invention will employ, unless otherwise indicated, conventional techniques of cell culture, molecular biology, microbiology, recombinant DNA, and immunology, which are within the skill of the art. Such techniques are explained fully in the literature. See, e.g., Maniatis, Fritsch and Sambrook, MOLECULAR CLONING: A LABORATORY MANUAL (1982); DNA CLONING, Volumes I and II (D.N. Glover, ed., 1985); OLIGONUCLEOTIDE SYNTHESIS (M.J. Gait ed., 1984); NUCLEIC ACID HYBRIDIZATION (B.D. Hames and S.J. Higgins, eds., 1984); B. Perbal, A PRACTICAL GUIDE TO MOLECULAR CLONING (1984); the series, METHODS IN ENZYMOLOGY (Academic Press, Inc.); VECTORS: A SURVEY OF MOLECULAR CLONING VECTORS AND THEIR USES (R.L. Rodriguez and D.T. Denhardt, eds., 1987, Butterworths); and J.H. Miller, EXPERIMENTS IN MOLECULAR GENETICS (1972, Cold Spring Harbor Laboratory), and HANDBOOK OF EXPERIMENTAL IMMUNOLOGY, Volumes I-IV (D.M. Weir and C.C. Blackwell, eds. 1986, Blackwell Scientific Publications).

All patents, patent applications, and publications mentioned herein, whether supra or infra, are hereby incorporated by reference.

The vaccines of the present invention are comprised of live avirulent Salmonella strains with an enhanced ability to colonize the intestinal tract of the immunized individual, and with an enhanced ability to induce immune responses in the individual that diminish the likelihood of colonization and persistence of heterologous Salmonella strains and prevent invasive diseases caused by Enterobacteriaceae, such as, for example, E. coli.

The Salmonella strains from which the strains used in the vaccines are constructed generally are avirulent due to mutation(s) in one or more genes which have "global regulation of pathogenicity", i.e., they co-ordinately regulate a number of genes including those that encode bacterial virulence factors. Examples of these "global mutant" strains are those which carry mutations, preferably deletion (open triangle) mutations delta-cya and delta-crp, which eliminate the ability to synthesize adenylate cyclase (ATP pyrophosphate lyase (cyclizing) EC 4.6.1.1) and the cyclic AMP receptor protein (CRP), respectively; methods for preparing these strains are known in the art (See, for example, Curtiss and Kelly (1987)). Strains of these mutants, Chi4064 and Chi4062, are on deposit with the American Type Culture Collection, and have been assigned Accession Nos. 53,648 and 53,647, respectively. Examples of "global mutants" are also mutants (preferably deletion mutants) in the phoP gene. phoP mutants of S. typhimurium are described in Galan and Curtiss (1989), and are on deposit with the American Type Culture Collection, and have accession numbers 53,864 and 53,866 for strains Chi3687 and Chi3689, respectively. Another strain which has a phoP::Tn10 mutation, wherein the Tn10 has inserted into the phoP gene is named Chi4126, and is on deposit with the ATCC under Accession no. __.

Several hundred wild-type strains of Salmonella representing over 40 species and obtained from a diversity of infected animal species are available for use. Many of these strains have been isolated from poultry and some of the strains available, especially S. enteriditis strains from Spain, England, and the United States, are known to have been transmitted from poultry to cause human disease. One or more of these strains may be used for the construction of the "global mutant" strains.

Although Salmonella, and particularly S. typhimurium is known to infect a wide diversity of animal species, there are differences in the ability of individual strains to infect and cause disease in various animal species. Thus, the Salmonella strain which is genetically-modified into a live avirulent vaccine strains is descended from a strain that is highly virulent for and has been passaged in the species, or immunologically related model species, of the individual to be immunized. For example, for vaccines for chickens, a genetically-modified live avirulent vaccine strain is descended from an S. typhimurium strain ARK101 (also called Chi3761), which has been passaged in chickens, and which has an oral LD₅₀ of approximately 2 x 10⁴ CFU. Chi3761 is a chicken-passaged derivative of ARK100 (also called Chi3663). A delta-cya delta-crp derivative of Chi3761, i.e., ARK106 (also called Chi3985), is tolerated at doses up to 1 x 10⁹ CFU without ill effect. Chi3985 when used to immunize mice induces high-level protective immunity to challenge with up to 1 x 10⁹ CFU of the parent Chi3761. A S. typhimurium strain, Chi4064, which is delta-cya delta-crp (Curtiss and Kelly (1987)), is highly virulent for mice, but is unable to kill one-day-old chicks by the oral route of inoculation. As shown in the Examples, this strain induces cross-protective immunity to caudal air sac challenge with 078:K80 E. coli. Another strain, Chi3985, also induces cross-protective immunity against challenge with E. coli strains capable of inducing colibacillosis. Another example of a virulent parental strain is ARK201 (also called Chi3850), which was isolated from a human who had received it as an egg-transmitted infection.

In another embodiment of the invention, the "global mutant" strain of Salmonella is further modified to enhance its ability to induce cross-protective immunity. One type of modification generates a phenotype in which the strain has the ability to produce wild-type LPS with multiple O-antigenic repeats when grown in culture medium with a suitable added carbohydrate, but fails to produce normal LPS upon infection into animal cells, whether the cells are in culture, or within an infected individual. Hence, when these mutants are used in the manufacture of vaccines, they are grown in culture medium containing the suitable added carbohydrate to produce wild-type LPS, and thus have a smooth phenotype, and to be sufficiently invasive to colonize the intestine and the GALT or BALT. However, after they have been used to immunize an individual and colonize the appropriate site, they gradually become rough and elicit a heightened immune response against exposed outer membrane proteins and the LPS core. Moreover, in the rough state they may be susceptible to nonspecific host defenses. In addition, the cells excreted should be both avirulent and nonimmunogenic, and should be less likely to survive in nature than a smooth Salmonella with wild-type levels of LPs. Mutation in either of two genes, galE or pmi, has the potential of conferring this phenotype on Salmonella.

The galE gene encodes UDP-galactose epimerase, which interconverts UDP-galactose with UDP-glucose, and permits cells grown on glucose to make UDP-galactose which is a precursor both for the LPS core and the O-antigen side chain in Salmonella. Strains with a mutation in the galE gene are unable to synthesize UDP-galactose when grown in media with glucose; therefore, they are unable to synthesize LPS and are rough, totally avirulent (Germanier and Furer (1971)), unable to invade through the mucin and glycocalyx lining the intestinal tract, and are extremely susceptible to nonspecific host defense mechanisms. galE mutants only make UDP-galactose when supplied with exogenous galactose. However, in mammalian cells it appears that most galactose is in a modified form (e.g., phosphorylated). Therefore, it is anticipated that in an mammalian or avian cell, a galE mutant would not make normal LPS, due to an insufficiency of galactose. Moreover, the remaining core in galE mutants is very similar to those of all enterics; therefore, an antibody response against this component of core may be more cross-protective against non-Salmonella enterics than an antibody response against the complete core.

The pmi gene encodes phosphomannose isomerase, which interconverts fructose-6-phosphate with mannose-6-phosphate. Growth of a pmi mutant in the presence of mannose allows normal synthesis of the Salmonella O-antigen side chain, whereas cultivation in medium containing glucose or another carbohydrate leads to the absence of the O-antigen side chain but a normal core polysaccharide.

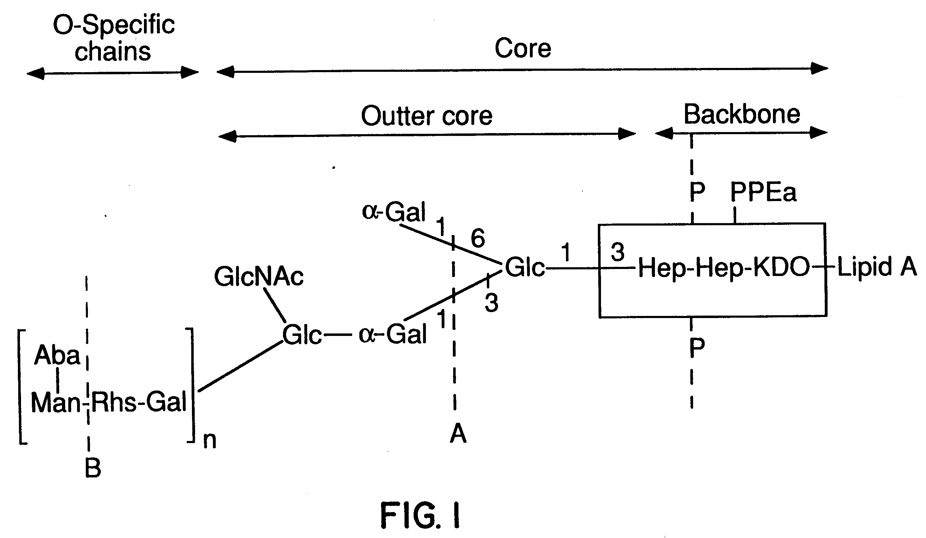

A schematic structure of the lipopolysaccharide of S. typhimurium is shown in Figure 1. The broken lines A and B indicate the points of termination of LPS synthesis in the following mutants: (A) UDP-galactose deficient (UDP-4-galactose-epimerase negative); (B) GDP-mannose deficient (phosphomannose isomerase).

Introduction of the galE mutation or pmi mutation into the "global mutant" strains may be accomplished by techniques known in the art, including for example, by transposition. In S. typhimurium, transposition may be accomplished using Tn10 transposons closely linked to galE or to pmi, for example, strains Chi3630 (Tn10 inserted into nadA and linked to galE) and Chi4149 (Tn10 linked to pmi). A generalized transducing phage may be used to transduce a galE or pmi mutation into a desired Salmonella strain, for example, one carrying a global mutation as described above, and the Tn10 eliminated by selection for fusaric acid resistance. The resulting strains should be reversibly rough, dependent upon whether galactose or mannose is included in the growth medium to permit synthesis of the LPS core and/or side chain. The delta-cya delta-crp S. typhimurium strains are able to grow on and ferment galactose and mannose, and have normal synthesis of LPS. Thus, galE and pmi derivatives are readily detectable by their inabilities to grow on or to ferment galactose and mannose, respectively.

In another embodiment of the invention, a "global mutant" strain of Salmonella, or derivative thereof, is further modified by a mutation (preferably a deletion) in the gene fur. The fur gene governs the synthesis of a repressor which, when iron is plentiful in the medium, prevents the expression of a number of genes including those encoding several outer membrane proteins (OMP)(Ernst et al. (1978)). In the absence of iron, the fur gene repressor does not block transcription of the iron-regulated genes; thus, allowing constitutive expression of all iron-regulated OMPs. Efficient iron chelation by Salmonella is probably not of critical importance when the microorganism is in the intestinal tract and the GALT, presumably because of adequate iron availability. Under these conditions, wild-type strains probably synthesize only low levels of iron-regulated OMPs; thus, these proteins probably could not serve as predominant antigens for an immune response. The use of a Salmonella fur mutation may enhance the immune response against the iron-regulated OMPs.

Iron sequestering is an important virulence attribute in E. coli strains capable of causing septicemia. Induction of an antibody response against iron-regulated OMPs may enhance the level of cross-protective immunity against E. coli, since it is known that substantial immunological cross-reactivity exists between the iron-regulated outer membrane proteins of E. coli and S. typhimurium. (See Chart and Griffiths (1985)).

Introduction of a mutation in the fur gene may be accomplished by techniques known in the art, including, for example, by transposition. Methods of detecting fur mutants are known in the art. For example, the Fur⁻phenotype of S. typhimurium is detected by the Arnow assay (1937). A method of creating fur::Tn10 mutations is described in the Examples, infra. A fur::Tn10 mutation may be introduced into a diversity of vaccine strains via P22HTint mediated transduction. The recipient strains may include, for example, those possessing delta-cya delta-crp mutations, and/or delta-phoP mutations, and/or additional mutations, e.g., pmi and/or galE. S. typhimurium fur mutants will constitutively express all iron-regulated outer membrane proteins, including the one which is homologous to the E. coli outer membrane protein which is capable of inducing passive protective immunnity against colibacillosis caused by E. coli in chickens.

Also contemplated within the invention are recombinant strains in which the live avirulent Salmonella vaccines serve as vectors for the expression of recombinant colonization and/or virulence antigens from other pathogens. In a preferred mode, the gene encoding the recombinant antigen is expressed in a balanced lethal host-vector system wherein the cloned gene is inserted into an Asd+ vector which is introduced into a delta-cya delta-crp delta-asd S. typhimurium strain such as chi4072 (ATCC accession number 67,538) or chi3987 (a delta-asd derivative of chi3985). A balanced lethal system comprising the recombinant Asd⁺ vector is described in WO/8903247. pmi or galE mutations can be introduced into chi4072 or chi3987 by methods described above. These strains will be particularly useful in expressing recombinant colonization and/or virulence antigens from other pathogens when these colonization and/or virulence antigens are exposed on the surface of the avirulent Salmonella cell. Thus, the recombinant vaccine strains can be grown in the presence of mannose or galactose to permit normal LPS synthesis so that upon oral administration to an animal to be immunized, they will colonize and invade the intestinal tract and GALT and then gradually lose the LPS from their surface to expose the expressed colonization and/or virulence antigens to the immune surveillance network and thus potentiate a heightened immune response.

Each of the terms in these embodiments of the invention is analyzed in the following discussion.

By vaccine is meant an agent used to stimulate the immune system of a living organism so that protection against future harm is provided. Immunization refers to the process of inducing a continuing high level of antibody and/or cellular immune response in which T-lymphocytes can either kill the pathogen and/or activate other cells (e.g., phagocytes) to do so in an organism, which is directed against a pathogen or antigen to which the organism has been previously exposed. Although the phrase "immune system" can encompass responses of uni-cellular organisms to the presence of foreign bodies, e.g., interferon production, in this application the phrase is restricted to the anatomical features and mechanisms by which a multi-cellular organism responds to an antigenic material which invades the cells of the organism or the extra-cellular fluid of the organism. The antibody so produced may belong to any of the immunological classes, such as immunoglobulins A, D, E, G or M. Of particular interest are vaccines which stimulate production of immunoglobulin A (IgA) since this is the principle immunoglobulin produced by the secretory system of warm-blooded animals, although vaccines of the invention are not limited to those which stimulate IgA production. For example, vaccines of the nature described herein are likely to produce a broad range of other immune responses in addition to IgA formation, for example, cellular and humoral immunity. Immune response to antigens is well studied and widely reported. A survey of immunology is given in Barrett, James T., Textbook of Immunology: Fourth Edition, C.V. Mosby Co., St. Louis, MO (1983).

A vertebrate is any member of the subphylum Vertebrata, a primary division of the phylum Chordata that includes the fishes, amphibians, reptiles, birds, and mammals, all of which are characterized by a segmented bony or cartilaginous spinal column. All vertebrates have a functional immune system and respond to antigens by producing antibodies. Thus all vertebrates are capable of responding to vaccines. Although vaccines are most commonly given to mammals, such as humans or dogs (rabies vaccine), vaccines for commercially raised vertebrates of other classes, such as the fishes and birds if of the nature described herein, are within the scope of the present invention.

In one embodiment of the invention is the use of an avirulent derivative of a pathogenic microbe that attaches to, invades and persists in the GALT or BALT as a carrier of the gene product which is used for stimulating antibody response against a pathogen or allergen. Avirulent does not mean that a microbe of that genus or species can not ever function as a pathogen, but that the particular microbe being used is avirulent with respect to the particular animal being treated. The microbe may belong to a genus or even a species that is normally pathogenic but must belong to a strain that is avirulent. By pathogenic is meant capable of causing disease or impairing normal physiological functioning. Avirulent strains are incapable of inducing a full suite of symptoms of the disease that is normally associated with its virulent pathogenic counterpart. Microbes as used herein include bacteria, protozoa, and unicellular fungi.

Techniques for transferring genetic material from a first organism to a second organism which normally does not exchange genetic material with the first organism, have recently become widely available as the result of rapidly expanding recombinant DNA technology. In this application, genetic material that has been transferred from one organism into a second in such a manner that reproduction of the second organism gives rise to descendants containing the same genetic material is referred to as a recombinant gene. The term gene is being used here in its broadest sense to represent any biological unit of heredity. It is not necessary that the recombinant gene be a complete gene as present in the parent organism, which was capable of producing or regulating the production of a macromolecule, for example, a functioning polypeptide. It is only necessary that the gene be capable of serving as the template used as a guide in the production of an antigenic product. The product may be one that was not found in that exact form in the parent organism. For example, a functional gene coding for a polypeptide antigen comprising 100 amino acid residues may be transferred in part into a carrier microbe so that a peptide comprising only 75, or even 10, amino acid residues is produced by the cellular mechanism of the host cell. However, if this gene product is an antigen that will cause formation of antibodies against a similar antigen present in the parent organism, the gene is considered to be within the scope of the term gene as defined in the present invention. Alternatively, if the amino acid sequence of a particular antigen or fragment thereof is known, it is possible to chemically synthesize the DNA fragment or analog thereof by means of automated gene synthesizers or the like and introduce said DNA sequence into the appropriate expression vector. At the other end of the spectrum is a long section of DNA coding for several gene products, one or all of which can be antigenic. Thus a gene as defined and claimed here is any unit of heredity capable of producing an antigen. The gene may be of chromosomal, plasmid, or viral origin.

In order for the gene to be effective in eliciting an immune response, the gene must be expressed. Expression of a gene means that the information inherent in the structure of the gene (the sequence of DNA bases) is transformed into a physical product in the form of an RNA molecule, polypeptide or other biological molecule by the biochemical mechanisms of the cell in which the gene is located. The biological molecule so produced is called the gene product. The term gene product as used here refers to any biological product or products produced as a result of the biochemical reactions that occur under the control of a gene. The gene product may be, for example, an RNA molecule, a peptide, or a product produced under the control of an enzyme or other molecule that is the initial product of the gene, i.e., a metabolic product. For example, a gene may first control the synthesis of an RNA molecule which is translated by the action of ribosomes into an enzyme which controls the formation of glycans in the environment external to the original cell in which the gene was found. The RNA molecule, the enzyme, and the glycan are all gene products as the term is used here. Any of these as well as many other types of gene products, such as glycoproteins and polysaccharides, will act as antigens if introduced into the immune system of an animal. Protein gene products, including glycoproteins and lipoproteins, are preferred gene products for use as antigens in vaccines.

In order for a vaccine to be effective in immunizing an individual, the antigenic material must be released in such a way that the immune system of the vaccinated animal can come into play. Therefore the live avirulent microorganism must be introduced into the animal. In order to stimulate a preferred response of the GALT or BALT cells as discussed previously, introduction of the microbe or gene product directly into the gut or bronchus is preferred, such as by oral administration, gastric intubation or in the form of aerosols, although other methods of administering the vaccine, such as intravenous, intramuscular, subcutaneous injection or intramammary or intrapenial or vaginal administration, is possible.

Recombinant DNA techniques are now sufficiently well known and widespread so as to be considered routine. In very general and broad terms, this method consists of transferring the genetic material, or more usually part of the genetic material, of one organism into a second organism so that the transferred genetic material becomes a permanent part of (recombines with) the genetic material of the organisms to which it is transferred. This usually consists of first obtaining a small piece of DNA from the parent organism either from a plasmid or a parent chromosome. A plasmid (also called an extrachromosomal element) is a hereditary unit that is physically separate from the chromosome of the cell. The DNA may be of any size and is often obtained by the action of a restriction endonuclease enzyme which acts to split DNA molecules at specific basepair sites. Following ligation to plasmid, phage or cosmid vectors to form recombinant molecules the recombinant molecules may be transferred into a host cell by various means such as transformation (uptake of naked DNA from the external environment, which can be artificially induced by the presence of various chemical agents, such as calcium ions). Other methods such as transduction are also suitable, wherein the recombinant DNA is packaged within a phage such as transducing phage or cosmid vectors. An additional means to introduce plasmid DNA into Salmonella is by electroporation. Techniques for introducing DNA into bacterial cells by electroporation are known in the art, and one such technique is described in the Examples. Once the recombinant DNA is in the carrier cell, it may continue to exist as a separate piece (generally true of complete transmitted plasmids) or it may insert into the host cell chromosome and be reproduced with the chromosome during cell division.

Derivatives of avirulent microbes are also contemplated to be within the scope of this invention. By derivative is meant sexually or asexually derived progeny and mutants of the avirulent strains including single or multiple base substitutions, deletions, insertions or inversions which retain the inability to produce functional adenylate cyclase and cAMP receptor protein with or without naturally occurring virulence plasmids. For example, strains such as Chi4062 and Chi4064 carry the gyrA mutation conferring nalidixic acid resistance which has been used herein as a convenient marker. However, drug resistance is not a desirable attribute for strains to be used as vaccines. Thus the gyrA mutation can be easily removed by transducing the gyrA⁺ (conferring sensitivity to nalidixic acid) gene into strains by selecting for inheritance of a closely linked Tn10 and then removing Tn10 by selection for fusaric acid resistance.

The dosages of recombinant or nonrecombinant avirulent Salmonella live vaccines required to elicit a protective immune response will vary with the antigenicity of a Salmonella gene product or cloned recombinant gene product and need only be a dosage sufficient to induce an immune response typical of existing vaccines. Routine experimentation will easily establish the required dosage. Typical initial dosages of vaccine could be 1 x 10⁷ to 1 x 10¹¹ CFU depending upon the size and age of the individual to be immunized. Administering multiple dosages can also be used as needed to provide the desired level of protective immunity.

The pharmaceutical carrier in which the vaccine is suspended in any solvent or solid or encapsulated in a material that is non-toxic to the inoculated animal and compatible with the carrier organism or antigenic gene product. Suitable pharmaceutical carriers include liquid carriers, such as normal saline and other non-toxic salts at or near physiological concentrations, and solid carriers not used for humans, such as talc or sucrose, also feed for farm animals. Adjuvants may be added to enhance the antigenicity if desired. When used for administering via the bronchial tubes, the vaccine is preferably presented in the form of an aerosol.

Immunization with a pathogen derived gene product can also be used in conjunction with prior immunization with the avirulent derivative of a pathogenic microorganism acting as a carrier to express the gene product specified by a recombinant gene from a pathogen. Such parenteral immunization can serve as a booster to enhance expression of the secretory immune response once the secretory immune system to that pathogen-derived gene product has been primed by immunization with the carrier microbe expressing the pathogen derived gene product to stimulate the lymphoid cells of the GALT or BALT. The enhanced response is known as a secondary, booster, or anamnestic response and results in prolonged immune protection of the host. Booster immunizations may be repeated numerous times with beneficial results.

### Deposits of Strains

A deposit of biologically pure cultures of the following strains were made with the American Type Culture Collection, 12301 Parklawn Drive, Rockville, Maryland. The accession number indicated was assigned after successful viability testing, and the requisite fees were paid. Access to said cultures will be available during pendency of the patent application to one determined by the Commissioner to be entitled thereto under 37 CFR 1.14 and 35 USC 122. All restriction on availability of said cultures to the public will be irrevocably removed upon the granting of a patent based upon the application. Moreover, the designated deposits will be maintained for a period of thirty (30) years from the date of deposit, or for five (5) years after the last request for the deposit; or for the enforceable life of the U.S. patent, whichever is longer. Should a culture become nonviable or be inadvertently destroyed, or, in the case of plasmid-containing strains, loose its plasmid, it will be replaced with a viable culture(s) of the same taxonomic description.

| Strain | Deposit Date | ATCC No. |
|---|---|---|
| Chi3761 | Nov. 3, 1989 | |
| Chi3985 | Nov. 3, 1989 | |
| Chi4126 | Nov. 3, 1989 | |
| Chi4137 | Nov. 3, 1989 | |
| Chi4152 | Nov. 3, 1989 | |

These deposits are for convenience only, and it is not to be construed that they are necessary to practice the invention.

### Examples

### Example 1

### Construction of S. typhimurium delta-crp delta-cya Strain chi3985

A wild-type, virulent S. typhimurium strain has been genetically modified by methods described in Curtiss and Kelly (1987). The strategy consists of mobilizing deletions of crp and cya genes that have been isolated and characterized in S. typhimurium SL1344 Chi3339 by placing the transposon Tn10 nearby the deletion (zhb::Tn10 linked to crp and zid::Tn10 linked to cya, respectively) and transducing the linked traits into a highly virulent S. typhimurium strain Chi3761 with selection for tetracycline resistance and a maltose-negative phenotype. Chi3761 was isolated from the spleen three days after oral infection of a one-day-old chick. Chi3761 has an oral LD₅₀ of 3 x 10³ CFU for one-day-old chicks. Transduction of the gene deletions with accompanying transposon (encoding tetracycline resistance) was facilitated by first making a high-titer bacteriophage P22HTint lysate, which packages the delta-crp-11 zhb::Tn10 or delta-cya-12 zid::Tn10 mutation into transducing particles. The resulting P22HTint lysate was then used to infect and transduce the genetic traits into another recipient Salmonella at a multiplicity of infection of 0.3. The phage-bacteria infection mixture was incubated for 10 min at 37^{o}C before 100 microliter samples were spread onto MacConkey agar (Difco Laboratories, Detroit, MI) containing 1% maltose (final concentration) supplemented with 12.5 micrograms tetracycline/ml. P22HTint propagated on Chi3773 (delta-crp-11 zhb::Tn10) was used to transduce the virulent strain Chi3761 to Mal⁻ Tet^{r}. After approximately 18 h incubation at 37^{o}C, transductants were picked and purified onto the same media. The resulting strain was designated Chi3828 and has the genotype delta-crp-11 zhb::Tn10. A culture of Chi3828 was diluted 1:10 into buffered saline with gelatin (BSG), 100 microliters spread onto fusaric acid-containing media (Maloy and Nunn, 1981) and incubated approximately 36 h at 37^{o}C. Fusaric acid-resistant colonies were picked and purified onto the same media, checked for loss of Tn10 (tetracycline sensitivity), P22HTint sensitivity and prototrophy, and the new strain was designated Chi3954 which has the genotype delta-crp-11 delta-[zhb::Tn10]. A culture of Chi3954 was then transduced with P22HTint propagated on Chi3670 to introduce the plasmid pSD110, which carries the wild-type crp+ gene from E. coli and ampicillin resistance. Selection was made on MacConkey agar + 1% maltose + 100 micrograms ampicillin/ml. An ampicillin-resistant Mal⁺ colony was picked and purified on the same media, checked for P22 sensitivity, and designated Chi3961 which has the genotype delta-crp-11 delta-[zhb::Tn10] pSD110+. A culture of Chi3961 was then transduced with P22HTint propagated on Chi3712 to introduce the delta-cya-12 and zid::Tn10 mutations. Selection was made on MacConkey agar + 1% maltose + 100 micrograms ampicillin/ml + 12.5 micrograms tetracycline/ml. An ampicillin resistant, tetracycline resistant, Mal⁻ colony was picked and purified onto the same media, checked for P22 sensitivity and designated Chi3962 which has the genotype delta-crp-11 delta-[zhb::Tn10] pSD110+ delta-cya-12 zid::tn10. A culture of Chi3962 grown in L broth containing 100 micrograms ampicillin/ml + 12.5 micrograms tetracycline/ml was diluted 1:10 into BSG, 100 microliter samples were spread onto fusaric-containing media and incubated approximately 36 h at 37^{o}C. Fusaric acid-resistant colonies were picked and purified onto the same media, checked for loss of Tn10 (tetracycline sensitivity), P22HTint sensitivity and prototrophy. Two out of ten colonies selected also lost the pSD110+ plasmid and one was designated Chi3985 which has the genotype delta-crp-11 delta-[zhb::Tn10] delta-cya-12 delta-[zid::Tn10] delta-cya-12 delta-[zid::Tn10]. The strain Chi3985 can be distinguished from its wild-type parent by the following phenotypic characteristics: the inability to ferment or grow on the carbon sources maltose, mannitol, sorbitol, melibiose, citrate, glycerol; decreased H₂S production; and decreased motility.

### Example 2

### Determination of the Comparative Virulence of Attenuated Mutants and Wild-Type Salmonella Strains

The attenuation of virulence in chickens of wild-type Salmonella strains by mutation in cya and/or crp was determined. The method for creating the attenuated strain were analogous to that described in Example 1, except that the indicated strains were substituted for the wild type strain in that example. The wild type strains are S. typhimurium Chi3306, Chi3663, Chi3761, and Chi3739, and S. enteritidis Chi3700. Chi3761 was isolated from the spleen of a chick orally inoculated three days earlier with Chi3663, a highly virulent S. typhimurium strain isolated from an infected horse. Chi3739 originated from 3860C, which was obtained from Robert C. Clarke, University of Guelph.

Bacteria for inoculation were grown as overnight standing cultures at 37^{o}C in L broth. These cultures were diluted 1:20 into prewarmed L brothand aerated at 37^{o}C for two to three hours until an optical density at 600 nm of about 0.8 to 1.0 was reached. The cells were concentrated 20-fold by centrifugation at 8000 x g for 10 min at room temperature followed by suspension in buffered saline plus gelatin (BSG). Fertile White Leghorn eggs (SPAFAS, Roanoke, IL) were incubated and hatched in Humidaire incubator-hatchers. Newly hatched chicks were given 100 microliters of the appropriate dilution of Salmonella via micropipette tip before being given food and water. Food and water were given to inoculated birds 30 minutes after infection. Birds were monitored daily for signs of disease (i.e., diarrhea, drooping, loss of appetite, weight loss, unresponsiveness and death). Infected birds were housed in modified guinea pig cages with filter bonnet tops, wire floors and thermostatically regulated temperatures in an animal room affording P2 level of containment. All materials leaving this room were autoclaved prior to further processing or dishwashing. The results of the study, which are presented in Table 1, demonstrate that a great diversity of delta-cya delta-crp mutants of S. typhimurium and S. enteritidis are avirulent for one-day-old chicks.

**Table 1**

| Virulence of Salmonella wild-type and attenuated mutants for orally inoculated one-day-old chicks | | | |
|---|---|---|---|
| Strain | Genotype | Origin | LD₅₀ (CFU) |
| | | | |

| A. S. typhimurium | | | |
|---|---|---|---|
| X3306 | gyrA1816 | SR-11 | >1 x 10⁹ |
| X4064 | delta-cya-1 | | |
| | delta-crp-2 gyrA1816 | X3306 | >1 x 10⁹ |
| X3663 | wild type | 30875 | 2 x 10⁴ |
| X3779 | delta-crp-10 | X3663 | >2 x 10⁹ |
| X3761 | wild type | X3663 | 3 x 10³ |
| X3784 | delta-crp-10 | X3761 | >5 x 10⁸ |
| X3954 | delta-crp-11 | X3761 | >2 x 10⁸ |
| X3962 | delta-cya-12 | X3761 | >3 x 10⁸ |
| X3985 | delta-crp-11 | | |
| | delta-cya-12 | X3954 | >4 x 10⁹ |
| X3739 | wild type | 3860C | 2 x 10⁵ |
| X3780 | delta-crp-10 | X3739 | >1 x 10⁹ |

| B. S. enteritidis | | | |
|---|---|---|---|
| X3700 | wild type | 4937 | 1 x 10⁷ |
| X3779 | delta-crp-10 | X3739 | >1 x 10⁹ |

### Example 3

### Construction of the phoP::Tn10 S. typhimurium Strain chi4126

The phoP gene of S. typhimurium regulates at least one of the nonspecific acid phosphatases and several other genes, one or more of which is important in virulence. A phoP::Tn10 strain was constructed.

Chi3688 is an S. typhimurium SL1344 strain with a phoP12 point mutation and a purB::Tn10 which is 90% cotransducible with the point mutation. A P22HTint lysate was made on this strain and the S. typhimurium LT2-Z strain Chi3000 was transduced to tetracycline resistance with the lysate. The transductants of this were screened for PhoP+ by the following methods. They were screened for the ability to cleave 5-bromo-4-chloro-3-indoyl phosphate (X-P); white colonies on X-P plates indicate a PhoP⁻ mutant. Also, X-P positive colonies were screened for production of nonspecific acid phosphatases by the method of Galan and Curtiss (1989); lack of orange colonies indicates the absence of these phosphatases, i.e., PhoP⁻. A PhoP+ purB::Tn10 transductant, called Chi4123, was further characterized for P22 sensitivity and ability to grow on minimal medium supplemented only with purines. A deletion (delta) mutation was created by selection for fusaric acid resistance, i.e., tetracycline sensitivity, and PhoP+, and was called Chi4124. Transduction of Chi4124 with P22HTint propagated on an S. typhimurium Tn10 library with simultaneous selection for PurB+ on minimal medium containing tetracycline selected for PurB+ transductants with closely linked Tn10 insertions. These were screened on X-P minimal medium for PhoP⁻, and for P22 sensitivity to give the phoP::Tn10 strain Chi4125. A P22HTint lysate was prepared on Chi4125 and the wild-type virulent S. typhimurium strain Chi3761 was transduced to tetracycline resistance and PhoP⁻ with the lysate. This phoP::Tn10 strain was called Chi4126. Deletion (delta) mutations can be made on this strain by selecting for fusaric acid resistance.

### Example 4

### Introduction of a galE Mutation into a delta-cya delta-crp Mutant of S. typhimurium

The generalized transducing phage P22HTint was propagated on Chi3630(Tn10 linked to galE) and was used to transduce the delta-cya delta-crp S. typhimurium strain Chi3985; selection for incorporation of Tn10 was by tetracycline resistance. The presence of the galE496 mutation was verified by the sensitivity of the cells to high concentrations of galactose (a property of strains with galE mutations), and resistance to bacteriophage P22 when grown on medium containing glucose (a phenotype associated with inability to make LPS). The resulting strain, Chi4136, was grown in medium with 0.05% galactose to permit normal LPS synthesis, and then transduced with a P22HTint lysate propagated on S. typhimurium LT-2 prototropic Chi3000. The transduction mixture was plated on minimal agar containing 0.5% glucose to select for NadA+ transductants. The resulting strain, Chi4137, was verified to be galactose-sensitive, rough, and P22 resistant when grown in the absence of galactose; it was also smooth and P22-sensitive when grown in medium with 0.05% galactose; and it still possessed the delta-cya delta-crp mutations.

### Example 5

### Introduction of a pmi Mutation into a delta-cya delta-crp Mutant of S. typhimurium

The Chi3985 pmi derivative strain was constructed by transducing Chi3985 with P22HTint propagated on Chi4149 (Tn10 linked to pmi). Transductants were selected for tetracycline resistance on MacConkey agar containing 1% mannose to screen for pmi cotransductants which are unable to ferment mannose. The resulting strain, Chi4151, was transduced with p22HTint propagated on Chi3000 with selection for a fusaric acid-resistant, tetracycline-sensitive derivative. The resulting strain, Chi4152 was verified to have a pmi mutation, to be tetracycline-sensitive, and to continue to possess the delta-cya delta-crp mutations.

### Example 6

### Introduction of a fur Mutation into a delta-cya delta-crp Mutant of S. typhimurium

The fur mutation in Chi3657 can be introduced into other strains by cotransduction with a Tn10 closely linked to the fur gene. Chi3627 possesses the nadA540::Tn10 insertion, and P22HTint propagated on Chi3627 has been used to transduce Chi3657 to tetracycline-resistance to yield strain Chi4130. The cotransduction frequency between fur and this Tn10 is about 10 percent. A Tn10 insertion on the other side of the fur gene is present in Chi3010. The propagation of P22HTint on this strain and transduction of Chi3657 yielded the strain Chi4131, which possesses a Tn10 to the left of fur and which is also co-transducible with fur at a frequency of 10 percent. P22HTint can be propagated on either Chi4130 or Chi4131 and used to transduce any S. typhimurium vaccine strain to tetracycline resistance fur, and the inserted Tn10 removed either by transduction or by selection for fusaric acid resistance.

An alternative method for generating fur mutations, caused by inactivation of the fur gene by insertion of Tn10, was accomplished by a related strategy. A fusaric acid-resistant derivative of Chi3627 was selected to eliminate Tn10 and to delete flanking sequences in the nadA gene. The strain Chi4132 possesses a delta-nadA mutation. Transduction of Chi4132 with P22HTint propagated on a S. typhimurium Tn10 library with simultaneous selection for NadA⁺ on minimal agar containing 0.5% glucose and tetracycline selected for Nad⁺ transductants with closely linked Tn10 insertions. In some instances, Tn10 inserted into the linked fur gene and inactivated it.

The Fur⁻ phenotype of S. typhimurium fur mutants is revealed by use of the Arnow assay (Arnow (1937)). In this assay, cells are grown in mineral salts minimal medium containing 10 micromolar FeCl₃ and 0.5 percent glucose. Cells are sedimented, and then to 0.5 ml of the culture supernatant fluid, the following is added in succession (with mixing between additions): 0.1 ml 5 M HCl, 0.5 ml molybdenum nitrate reagent (prepared by adding 1 g sodium molybdate to 1 g sodium nitrate in 5 ml deionized distilled water), followed by 0.1 ml 10 N NaOH. Constitutive siderophore synthesis by fur mutants is quantitated by reading the absorbancy at 515nm.

### Example 7

### Detection of LPS Synthesis or Loss in galE or pmi Mutant S. typhimurium Strains Growing in Culture

Several strains of S. typhimurium grow in Chinese Hamster Ovary (CHO) cells with generation times of three to four hours. CHO cells are grown in Eagle's minimal essential medium supplemented with 10% (vol/vol) fetal calf serum (FCS), penicillin (100U/ml) and streptomycin (100 micrograms/ml) and infected with bacteria while in Hank's balanced salt solution (HBSS). These media are devoid of added galactose or mannose. Thus, galactose or mannose required for synthesis of LPS by galE or pmi mutants would have to result from endogenous synthesis of these substrates by the CHO cells.

The Chi4137 (galE) or Chi4152 (pmi) derivative mutants are grown in L broth medium lacking glucose, but containing 0.05% galactose or 0.5% mannose and 300 mM NaCl. This medium enhances the expression of S. typhimurium inv genes, whose regulation is dependent upon osmolarity. Infection of CHO cell monolayers is accomplished by inoculating them with S. typhimurium cells at a multiplicity of infection of 10 bacteria per cell. After 2 hours in HBS, the monolaylers are washed in HBSS and then incubated with Eagle's minimum essential medium (MEM) containing gentamicin (100 micrograms/ml) to eliminate extracellular bacteria. Control studies are performed using the wild-type Chi3761 strain, and pmi and galE derivatives of Chi3761. Samples of CHO cells are taken at consecutive periods after attachment and invasion. The cells are lysed by addition of 0.1% sodium deoxycholate in PBS, chilled on ice, and the S. typhimurium cells recovered by the methods described by Finlay et al. (1989). The recovered cells are counted, the amount of protein determined, and LPS fractions quantitated by the Limulus Assay (Tanamoto and Homma (1982)), and by densitometry of silver stained LPS fractions after gel electrophoresis (Tsai and Frasch (1983)). Western immunoblotting is also a very sensitive assay by which the differential rates of synthesis of LPS core and LPS side chains may be distinguished.

If galactose and mannose are limiting in CHO cells, the total LPS side-chains will show little increase, whereas the amounts of S. typhimurium protein and LPS core should increase significantly (about 8- to about 32-fold during a 24-hour period).

### Example 8

### Induction by galE and pmi Mutants of Cross-Protective Immunity to Colonization by Homologous and Heterologous Salmonella Serotypes

The vaccine strains which are compared are Chi3985, which has delta-cya delta-crp mutations, and derivatives of Chi3985 which have a pmi mutation (i.e., Chi4152) or a galE mutation (i.e., Chi4137). Chicks are immunized with these strains at one day and at three days of age, and then challenged two and four weeks later with either a derivative of Chi3761 possessing a mutation to rifampicin resistance (rpoB1911), or with a derivative of of the wild-type S. enteriditis phage type 4 strain, i.e., Chi3850, which would also have the rpoB1911 allele. The rpoB1911 mutation is without effect on the virulence of S. typhimurium or S. enteriditis. Since Chi3761 and its derivatives with the galE and pmi mutations are antibiotic sensitive, a Tnmini-tet marker is inserted into the virulence plasmid by transduction with the bacteriophage P22HTint propagated on Chi3456. (See Gulig and Curtiss (1987) for the method of inserting the marker into the plasmid). This marker permits differential quantitation of the vaccine and challenge strains in feces or at necropsy.

Chicks are perorally immunized at one day and three days of age with 1 x 10⁹ CFU of either Chi3761, or Chi4152 (grown in L Broth containing 0.5% mannose), or Chi4137 (grown in L Broth containing 0.05% galactose). At two and four weeks after peroral immunization, groups of five birds are perorally inoculated with 1 x 10², or 1 x 10³, or 1 x 10⁴ of either the rifampicin-resistant (Rif^{r}) derivative of S. typhimurium, Chi3761, or the Rif^{r} derivative of S. enteritidis, Chi3850. Fecal specimens are collected daily from the inoculated birds, and measured aliquots are used to quantitate the S. typhimurium or S. enteriditis challenge strain present in the specimen. Quantitation is by plating on MacConkey agar with 1% (final concentration) lactose and 50 micrograms rifampicin per ml.

In the event that titers are too low to detect by these means, the presence or absence of the challenge strain may be detected by incubating overnight at 37^{o}C an aliquot of the fecal matter in Selenito broth (Liefson (1936)) containing 50 micrograms rifampicin/ml, and then scoring for the presence of Rif^{r} Chi3761 or Chi3850 cells. The presence of the vaccine strain is detected by plating on Difco MacConkey agar (Difco Laboratories, Detroit, MI) with 1% maltose and 12.5 micrograms/ml tetracycline. Unimmunized chicks are used as controls to determine the minimum titers of Rif^{r} Chi3761 and Chi3850 to cause colonization, persistent infection and shedding.

Two and four weeks after challenge with the wild-type strains, birds are sacrificed and the titers of the wild-type challenge and vaccine strains in the spleen, contents of the small intestine, cecum, and large bowel are quantitated by plating aliquots of the samples on MacConkey agar with 1% lactose and rifampicin, or MacConkey agar with 1% maltose and tetracycline. The Rif^{r} challenge strain, which is Mal+ Tc^{s} Lac⁻ Rif^{r}, grows only on the first medium, and the vaccine strains, which are Mal⁻ Tc^{r} Lac⁺ Rif^{s}, grow only on the second medium.

### Example 9

### Effect of Constitutive Expression of Iron-Regulated OMPs by S. tymphimurium on Growth in Mammalian Cells

Derivatives of S. typhimurium Chi3761 and Chi3985 which contain the fur::Tn10 mutation are used in these studies; controls for the study are the Chi3761 and Chi3985 parental cells. The CHO cells serve as a model for vertebrates in measuring the invasiveness and viability of the fur mutant strain. Growth of CHO cells is as described supra. The S. typhimurium are grown in L broth containing 0.1% glucose and 300 mM NaCl. After sedimentation and resuspension in buffered saline, the cells are used for attachment to and invasion of CHO cells in culture, as described above. Periodically, cells are resuspended, lysed, and the number of intracellular S. typhimurium cells is determined.

### Example 10

### Effect of Constitutive Expression of Iron-Regulated OMPs by S. tymphimurium on Virulence in Chicks

The Chi3761 fur::Tn10 strain and its Chi3761 parent strain are grown in L broth containing 0.1% glucose and 300 mM NaCl, as described above. One-day old chicks are orally inoculated with 100 microliters of bacterial suspension in buffered saline. Micropipettes are used to inoculate groups of five one-day old chicks with either 1 x 10³ CFU, 5 x 10³ CFU, or 2.5 x 10⁴ CFU. Dead chicks are posted to verify that they died of septicemia caused by S. typhimurium.

Sera is collected from birds that survive three to four weeks after challenge so that titers of antibodies directed against iron-regulated proteins can be determined. Antibody titration is done by the ELISA method and/or by western blot analysis to proteins produced by a Fur⁻ S. typhimurium strain. Reactivity of sera with outer membrane protein fractions of the 078:K80 E. coli strain Chi7122 grown in iron and under iron limitaiton can also be quantitated. The degree of reactivity to antibody in Western blots is quantitated using a Molecular Dynamics densitometer.

### Example 11

### Induction by fur::Tn10 Mutants of Cross-Protective Immunity to Colonization by Homologous and Heterologous Salmonella Serotypes

These studies are similar to those done with the galE and pmi mutants, except that the derivative strain carries a fur::Tn10 mutation which allows the constitutive expression of iron-regulated OMPs. The fur::Tn10 mutation is introduced into the parental strains, both virulent and avirulent, by P22HTint transduction. Colonization of the wild-type Rif^{r} S. typhimurium and S. enteritidis in respective groups of immunized birds is determined by quantitating viable Salmonella in fecal samples, as described above.

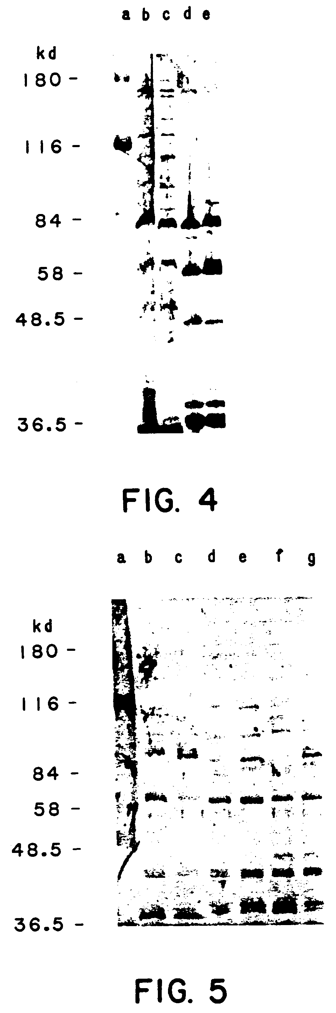

The induction and the cross-reactivity of the iron-regulated OMPs of E. coli and of Salmonella were examined. Figure 4 shows the results of a Western immunoblot of whole bacterial extracts run on a 7.5% SDS-polyacrylamide gel, transferred to nitrocellulose and probed with serum from a rabbit immunized with formalin-killed 078:K80 E. coli strain Chi7122 grown in tryptic soy broth. The lanes contained the following: lane a, prestained molecular weight markers; lane b, Chi7122 grown in tryptic soy broth with high iron (10 micromolar FeCl₃; lane c, Chi7122 grown in tryptic soy broth with low iron (300 alpha,alpha'-dipyridyl); lane d, S. typhimurium Chi4064 grown in tryptic soy broth with high iron; and lane e, Chi4064 grown in tryptic soy broth with low iron.

Figure 5 shows the results of a Western immunoblot of whole bacterial extracts run on a 7.5% SDS-polyacrylamide gel, transferred to nitrocellulose and probed with a rabbit antiserum obtained from a rabbit immunized twice with an outer membrane preparation from the 078:K80 E. coli strain Chi7122 grown in tryptic soy broth with low iron (300 micromolar alpha, alpha' dipridyl) for expression of iron-regulated outer membrane proteins. The contents of the lanes were as follows: lane a, prestained molecular weight markers; lane b, Chi7122 grown in tryptic soy broth with high iron; lane c, Chi7122 grown in tryptic soy broth with low iron; lane d, S. typhimurium RB18, a fur mutant, grown in tryptic soy broth with high iron; lane e, RB18 grown in tryptic soy broth with low iron; lane f, S. typhimurium LT-2 Chi3000 grown in tryptic soy broth with high iron; and lane g, Chi3000 grown in tryptic soy broth with low iron. The arrows mark the position in the gel of iron-regulated outer membrane proteins that are evident in lanes c, d, e and g.

### Example 12

### Oral Immunization of Chickens with a delta-cya delta-crp S. typhimurium Strain: Protective Effect Against Colonization by Virulent Wild-Type S. typhimurium Strain

In this study, the immunizing and challenge strains are grown to log phase, sedimented and suspended in buffered saline with gelatin. Oral inoculation is with 100 microliter samples of the S. typhimurium strains.

Three day old chicks are immunized with 1 x 10⁹ cells of S. typhimurium strain Chi3985, which is a delta-cya delta-crp mutant. At five weeks of age, both immunized and unimmunized chickens are orally challenged with 1 x 10⁶ cells of the virulent strain, Chi3761. Titers of excreted cells are quantitated by plating on Brilliant Green Agar (which permits detection at 1 x 10²/cc). The absence of shedding is monitored by the inability to detect Chi3761 cells by selenite broth enrichment. The results, which are presented in Table 2, show that immunization with the delta-cya delta-crp vaccine strain significantly reduced initial colinization and duration or persistence of the invasive virulent challenge strain.

**Table 2**

| Oral immunization of chickens with a delta-cya delta-crp S. typhimurium strain to protect against colonization by virulent wild-type S. typhimurium | | | |
|---|---|---|---|
| Immunization given at | Time after challenge | Excretors/total | Mean Titer excreted (Log₁₀ CFU) |
| none | 12 hours | 11/11 | 8.0 |
| none | 1 week | 11/11 | 4.3 |
| none | 2 weeks | 8/11 | 4.3 |
| none | 4 weeks | 8/10 | 1.8 |
| none | 5 weeks | 8/10 | 2.3 |
| 3 days | 12 hours | 6/6 | 4.2 |
| 3 days | 1 week | 6/6 | 2.2 |
| 3 days | 2 weeks | 1/6 | 0.7 |
| 3 days | 4 weeks | 2/6 | 0.3 |
| 3 days | 5 weeks | 1/6 | 0.2 |

### Example 13

### Cross Protective Immunity to Virulent E. coli Induced by a phoP::Tn10 Strain of S. typhimurium

One-day old chicks are orally inoculated with S. typhimurium strain Chi4126, which is a phoP::Tn10 mutant strain. Two weeks later, survivors are challenged with the O78:K80 virulent E. coli strain Chi7122. Challenge is by injection of 7.3 x 10⁵ CFU into the right caudal air sac. The wild type parent of Chi4126 has an oral LD₅₀ of 3 x 10³ CFU in one-day old chicks. The LD₅₀ of Chi7122 in two-week old chickens is 5 x 10⁴ CFU. The results of the study, shown in Table 3, demonstrate the relative avirulence of the phoP::Tn10 S. typhimurium strain, and also demonstrate that immunization with this strain has the ability to protect against infection by virulent E. coli.

**Table 3**

| Avirulence of An Orally Administered phoP::Tn10 S. typhimurium Mutant, and Induction of Cross-Protective Immunity to Challenge with Virulent E. coli | | | |
|---|---|---|---|
| Chi4126 inoculating dose (CFU) | Survival live/total | Chi7122 challenge dose (CFU) | Survival live/total |
| none | -- | 7.3 x 10⁵ | 0/3 |
| 9 x 10⁴ | 3/4 | 7.3 x 10⁵ | 2/3 |
| 9 x 10⁵ | 2/4 | 7.3 x 10⁵ | 2/2 |
| 9 x 10⁶ | 3/4 | 7.3 x 10⁵ | 3/3 |
| 9 x 10⁷ | 3/4 | 7.3 x 10⁵ | 3/3 |

### Example 14

### Protection by Oral Immunization with S. typhimurium delta-cya delta-crp Strains Against Challenge with Virulent O78:K80 E. coli

Two delta-cya delta-crp S. typhimurium strains, Chi4064 and Chi3985, which were shown to be avirulent in chickens, are utilized to orally immunize separate groups of one-day-old chickens with 10⁹ bacteria. Immunized chickens and control groups are challenged with E. coli O78:K80:H9 strain Chi7122, which causes air sacculitis, pneumonia, and septicemia, and which has an LD₅₀ of 4 x 10⁴ CFU in two-week old chickens, and 7 x 10⁵ CFU in four-week old chickens. Challenge is by injection of E. coli into the right caudal air sac.

The results, presented in Table 4, showed that two weeks after immunization with either strain, birds were protected against 10X to 100X the LD₅₀. Four weeks after immunization, birds were protected up to 100X the LD50. Approximately half of unimmunized controls survived challenge with 1X the LD₅₀; none of these controls survived higher challenge doses. The LD₅₀ of Chi7122 inoculated into the caudal air sac is 4 x 10⁴ CFU and 7 x 10⁵ CFU in two-week-old and four-week-old chickens, respectively. In the Table, the symbol (*) indicates that the chickens were orally immunized when three days old.

**Table 4**

| Protection of Chickens Orally Immunized with S. typhimurium delta-cya delta-crp Vaccine Strain Against Challenge with Virulent E. coli | | | | |
|---|---|---|---|---|
| Immunizing strain | Immunizing dose (CFU) | Age at Challenge | Challenge dose (CFU) | Survival live/total |
| none | none | 2 weeks | 6.1 x 10⁶ | 0/4 |
| Chi4064 | 1.2 x 10⁹ | 2 weeks | 6.1 x 10⁶ | 2/4 |
| none | none | 2 weeks | 2.8 x 10⁵ | 0/4 |
| Chi3985 | 1.3 x 10⁹ | 2 weeks | 2.8 x 10⁵ | 4/4 |
| Chi3985 | 1.3 x 10⁹ | 2 weeks | 2.8 x 10⁶ | 2/4 |
| none | none | 2 weeks | 7.4 x 10⁵ | 2/5 |
| Chi3985* | 3.8 x 10⁹ | 2 weeks | 7.4 x 10⁵ | 4/5 |
| Chi3985* | 3.8 x 10⁹ | 2 weeks | 7.4 x 10⁶ | 2/4 |
| none | none | 4 weeks | 3.5 x 10⁸ | 1/4 |
| Chi4064 | 5.2 x 10⁹ | 4 weeks | 3.5 x 10⁸ | 5/5 |
| none | none | 4 weeks | 6.6 x 10⁷ | 0/5 |
| Chi4064 | 8.2 x 10⁹ | 4 weeks | 6.6 x 10⁷ | 4/5 |
| none | none | 4 weeks | 1.6 x 10⁶ | 2/5 |
| Chi3985* | 3.8 x 10⁹ | 4 weeks | 1.6 x 10⁶ | 4/5 |
| Chi3985* | 3.8 x 10⁹ | 4 weeks | 1.6 x 10⁷ | 2/4 |

Within an hour after inoculation into the caudal air sac, Chi7122 is detectable in blood. In order to investigate whether immunization of one-day-old chicks with the delta-cya delta-crp S. typhimurium vaccine strain Chi3985 reduced the ability of E. coli to enter and/or survive in the circulatory system, studies were conducted with one-day-old chicks that were perorally immunized with 6.4 x 10⁹ CFU of S. typhimurium Chi3985 (control chicks were unimmunized). Two weeks later, both groups were challenged with 10 LD₅₀s of the virulent E. coli strain Chi7122 by injection into the caudal air sac. At each time point, 100 microliters of blood was removed by venipuncture into the wing vein and the numbers of bacteria enumerated. The data in Table 5 indicate that immunization of one-day-old chicks with Chi3985 causes marked reduction in bacteremia and precludes septicemia when challenged two weeks later. In the table, the symbol (b) indicates that two birds died before day 4; the average number is for the remaining two birds.

**Table 5**

| Average number of E. coli in the blood as a function of Time after caudal air sac challenge with virulent chi7122 in immunized and unimmunized birds^{a} | | | | | | | | |
|---|---|---|---|---|---|---|---|---|
| Treatment | 1h | 2h | 3.5h | 5h | 1day | 2day | 3day | 4day |
| Immunized | 0.3 | 1.5 | 5.3 | 15.8 | 35.5 | 42.5 | 3.3 | fine |
| Unimmunized | 0.3 | 1.0 | 12.8 | 51.0 | 1173 | 5900 | 620^{b} | dead |

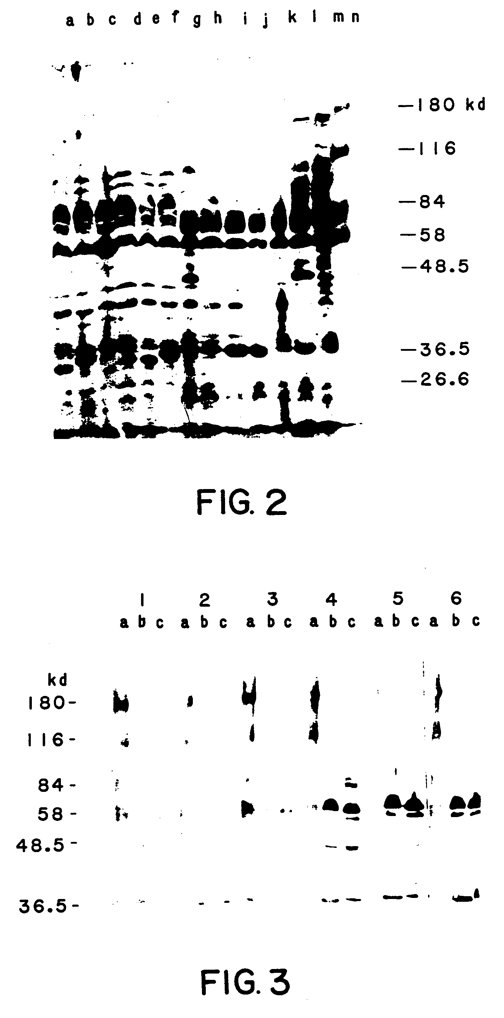

S. typhimurium vaccine strain Chi4062 is able to elicit rapid production of activated respiratory phagocytic cells to provide a non-specific means for resistance to E. coli infection shortly after immunization. However, the survival to caudal air sac challenge with E. coli Chi7122 two to four weeks after oral immunization with Chi4064 or Chi3985 is probably due to a different mechanism. Therefore, in order to determine whether there was a basis for the ability of S. typhimurium vaccine strains to induce cross-protective immunity against various species of Enterobacteriaceae, E. coli and Salmonella protein antigens were separated on SDS gels and reacted with various preimmune, absorbed and unabsorbed antisera against E. coli Chi7122 raised in rabbits, and against S. typhimurium Chi3306 (the parent to Chi4064) raised in rats. The results are shown in Figure 2, which is a Western immunoblot of whole bacterial extracts which were run on a 7.5% SDS-polyacrylamide gel, transferred to nitrocellulose, and probed with serum from a rat injected i.p. with 10⁶ viable Chi3306 cells, boosted twice (4 and 6 weeks later) with 10⁷ viable Chi3306 cells, and then bled two weeks after the last immunization. Serum was stored in 50% glycerol at -20^{o}C. In the figure, the lane contents are as follows: lane a, Chi7027 E. coli 02a:K-:H5 (turkey); lane b, Chi7010 E. coli 036:K (turkey); lane c, Chi7011 E. coli 0143:K-:H27 (turkey); lane d, Chi7112 E. coli 01:K1:H7 (human septicemia); lane e, Chi7110 E. coli 01:K1 (human UTI); lane f, Chi7122 E. coli 078:K80:H9 (chicken); lane g, Chi3663 S. typhimurium (horse); lane h, Chi3700 S. enteritidis (human); lane i, Chi3202 S. albany (human); lane j, Chi3214 S. infantis (human); lane k, Chi3210 S. hadar (human); lane l, Chi3749 S. heidelberg (chicken); lane m, Chi3750 S. agona (chicken); and lane n, prestained molecular weight markers.

The results in Figure 2 reveal that antibodies against S. typhimurium raised in rats react with a substantial number of proteins present in a diversity of Salmonella species and in various E. coli which are causitive of various disease states in individuals of a variety of species, including mammals and birds. The reciprocal is true in that antibodies raised against E. coli proteins also react with proteins from many different E. coli strains as well as against a diversity of Salmonella species. Thus, the results establish a basis for the ability of S. typhimurium vaccine strains to induce cross-protective immunity against various species in the Enterobacteriaceae.

In order to establish that the cross-protective effect of immunization with the Salmonella vaccine strains resulted from the immunogenicity of the strains, the titers of circulating antibodies in sera from birds immunized with Chi3985, which react with surface proteins in both E. coli Chi7122 and in the vaccine strain Chi3985 was examined. Figure 3 shows Western immunoblots of whole bacterial extracts which were run on a 7.5% SDS polyacrylamide gel, transferred to nitrocellulose, and probed with serum from either immunized or unimmunized (i.e., control) three-week-old chickens. Blots one, two and three are probed with serum from three unimmunized chickens. Blots four, five, and six are probed with serum from three birds immunized at one day of age. Immunization was perorally with 1 x 10⁹ CFU of the delta-cya delta-crp S. typhimurium strain Chi3985. In the blots, lane a contains prestained molecular weight markers; lane b contains E. coli Chi7122 (078:K80:H9) grown in broth; and lane c contains S. typhimurium Chi3985 grown in broth.

The results in Figure 3 demonstrates that the sera from birds immunized with Chi3985 contains high titers of circulating antibodies that react with surface proeins in both E. coli Chi7122 and in the vaccine strain Chi3985. These antibodies were not detected in sera from unimmunized birds.

### Example 15

### Evaluation of galE Mutation or pmi Mutation or fur Mutation on Protection by Oral Immunization with S. typhimurium delta-cya delta-crp Strains Against Challenge with Virulent 078:K80 E. coli

It was demonstrated in Example 14 that immunization of one-day old chicks with 1 x 10⁹ CFU of a delta-cya delta-crp strain of S. typhimurium induced increasing immunity to caudal airsac challenge with an O78:K80 E. coli strain. The effect of the inclusion of mutations in galE, or pmi, or fur on the induced immunity is examined as follows.

White Leghorn chicks at one day and three days of age are orally immunized with 1 x 10⁹ cells of vaccine strains grown in L broth with 0.1% glucose, L-broth with 0.05% galactose (for galE strains), and L broth with 0.1% mannose (for pmi strains) and 300 mM NaCl. Five vaccine strains are compared. These include the delta-cya delta-crp S. typhimurium vaccine strain Chi3985 and four Chi3985 derivatives with pmi (Chi4152), pmi and fur, galE (Chi4137), and galE and fur mutations.

Groups of five birds are immunized with 1 x 10⁹ CFU of each vaccine strain. The vaccine strains are grown in L broth supplemented with the appropriate carbohydrate, 300 mM NaCl, and after harvesting, are resuspended in buffered saline with gelatin. Groups of immunized birds are challenged with 1 x 10⁶, 1 x 10⁷, and 1 x 10⁸ CFU of Chi7007 E. coli O78:K80 two and four weeks after immunization. Before challenge, the challenge strain is grown in tryptic soy broth, harvested, and suspended in phosphate buffered saline (PBS). Challenge is by the inoculation of 100 microliters of the suspension into the caudal air sac. Groups of unimmunized birds are used as controls. Birds which die are posted to verify that they have succumbed due to E. coli infection. Verification is by the presence of typical fibrous deposits characteristic of airsaculitis, pericarditis, pneumonia, etc.

### Example 16

### Introduction of plasmid DNA into Salmonella by Electroporation

An means to introduce plasmid DNA into Salmonella is by electroporation. A 10 ml L broth grown culture of the S. typhimurium recipient strain is grown to log phase (i.e., to an absorbance at 600 nm of 0.5 to 0.8). Cells are chilled on ice for 15 to 30 min, sedimented by centrifugation at 5000 x g for 15 min at 4^{o}C. The pellet is suspended in 10 ml ice cold 1 mM HEPES, pH 7 buffer. The centrifugation and resuspension in HEPES is repeated after which the cells are sedimented and suspended in 1.25 ml of 10% glycerol. Cells are further concentrated by centrifugal sedimentation and suspended finally in 125 microliters of 10% glycerol at a cell concentration of approximately 3 x 10¹⁰ cfu/ml. Forty ml of cells on ice are mixed with 5 to 10 microliters of DNA suspended in Tris-EDTA, pH 8, buffer which is mixed and allowed to stand on ice for approximately 1 min. The Gene Pulsar apparatus is set at 25 microF and 2.5 kV with pulse controller at 200 o. The cell-DNA mixture is transferred to a cold 0.2 cm electroporation cuvette. The suspension is shaken to the bottom of the cuvette and the cuvette placed in the base of the chamber. A single pulse is given for 4.5 to 5 msec duration after which the cuvette is removed from the chamber and 1 ml of SOC medium (containing 2% Bacto-tryptone, 0.5% Bacto-yeast, 10 mM NaCl, 2.5 mM KCl, 10 mM MgSO₄, and 20 mM glucose) is added to the cuvette to quickly suspend the cells using a Pasteur pipette. The cell suspension is then transferred to a glass tube and incubated at 37^{o}C with rapid aeration for one h. Samples of 100 microliters of a 1:10 dilution and 100 microliters undiluted are plated on appropriate selective medium such as MacConkey agar containing 1% maltose + 100 micrograms ampicillin/ml for introducing the crp⁺ plasmid pSD110 into a strain with a delta-crp mutation.

### Example 17

### Evaluation of Ability of Unimmunized Cage Mates to BecomeImmunized by Immunized Chickens

The S. typhimurium vaccine strains delta-cya delta-crp pmi and delta-cya delta-crp galE are reversibly rough and can only synthesize LPS core and side chains when provided with an exogenous supply of mannose or galactose, respectively. These sugars are not likely to be abundantly present in intestinal contents. Therefore, S. typhimurium strains with pmi or galE mutations should colonize the intestine for a shorter duration than their wild-type parents and should be excreted in a rough form which should be both avirulent and nonimmunogenic.

The vaccine strain Chi3985 (delta-cya delta-crp) and its pmi or galE derivatives, Chi4152 and Chi4137, respectively, are grown in L broth with appropriate carbohydrates to permit LPS synthesis, and in the presence of 300 mM NaCl to enhance invasiveness. The resulting cells are suspended in buffered saline plus gelatin and used to perorally inoculate one-day-old chicks. Groups of five chicks immunized with each strain are each housed with a group of five unimmunized chicks. Birds are wing-banded in order to collect data on individuals. Cloacal swabs are used to quantitate the level and duration of excretion, and to determine whether non-immunized birds become colonized. At four weeks of age, all birds are bled and titers of antibody against Salmonella LPS and outer membrane protein antigens are determined by an ELISA method.

### Industrial Applicability

Vaccines which are comprised of avirulent strains of Salmonella which are able to induce immunity to homologous and heterologous Salmonella serotypes and to other gram-negative enteric bacteria are useful in treating mammals and birds to ameliorate the effect of disease in populations exposed to virulent strains of these gram-negative bacteria. Included in this population are mammals, and in particular birds, which are not only often infected with these pathogenic bacteria, but which also are in the chain of transmission to humans.

Strains of avirulent Salmonella are described supra. which possess at least one mutation in a gene which globally regulates other genes, and which possess, in addition at least one other mutation of the following type: a mutation either in a gene encoding an enzyme in lipopolysaccharide synthesis, which results in a reversibly rough phenotype; or in a gene which regulates the synthesis of iron-regulated OMPs, such that the mutation leads to constitutive expression of these proteins. These strains are useful in the production of the aforementioned vaccines.

## Claims (Claims for the following Contracting State(s): AT, BE, CH, LI, DE, DK, FR, GB, IT, NL, SE)

1. A vaccine for treatment of an individual for infections by gram-negative bacteria comprised of live avirulent *Salmonella* which are able to induce immunity to homologous and heterologous *Salmonella* serotypes and to other gram-negative enteric bacteria, wherein the *Salmonella* possess a mutation in a gene which encodes adenylate cyclase (*cya*) and/or cyclic AMP receptor protein (*crp*), and also possess a mutation in a gene encoding an enzyme in lipopolysaccharide synthesis which results in a reversibly rough phenotype, the amount of said live cells being sufficient to improve the resistance of the individual to infection by the gram-negative enteric bacteria, the *Salmonella* cells being present in a pharmaceutically acceptable carrier.

2. A vaccine according to claim 1, wherein the avirulent *Salmonella* possess a mutation in a *galE* gene that encodes UDP-galactose epimerase.

3. A vaccine according to claim 1, wherein the *Salmonella* possess a mutation in a *pmi* gene that encodes phosphomannose isomerase.

4. Use of live avirulent *Salmonella*, which possess at least one mutation in a gene involved in the global regulation of pathogenicity of the *Salmonella* and also possess a mutation in a gene encoding an enzyme in lipopolysaccharide synthesis which results in a reversibly rough phenotype, for the manufacture of a medicament for use in inducing immunity to heterologous *Salmonella* serotypes or to gram-negative bacteria other than *Salmonella*.

5. Use according to claim 4, wherein the *Salmonella* possess a mutation in a *phoP* gene that regulates genes that allow the *Salmonella* to survive in macrophages.

6. Use according to claim 4, wherein the *Salmonella* possess a mutation in a *galE* gene that encodes UDP-galactose epimerase.

7. Use according to claim 4, wherein the *Salmonella* possess a mutation in a *pmi* gene that encodes phosphomannose isomerase.

8. A vaccine for treatment of a individual for infections by gram-negative bacteria comprised of live avirulent *Salmonella* which are able to induce immunity to homologous and heterologous *Salmonella* serotypes and to other gram-negative enteric bacteria, wherein the *Salmonella* possess at least one mutation in a gene involved in the global regulation of pathogenicity of the *Salmonella*, and also possess a mutation in a gene regulating the synthesis of iron-regulated outer membrane proteins (OMP), such that the mutation leads to constitutive expression of iron regulated OMP, the amount of said live cells being sufficient to improve the resistance of the individual to infection by the gram negative enteric bacteria, the *Salmonella* cells being present in a pharmaceutically acceptable carrier.

9. A vaccine according to claim 8, wherein the avirulent *Salmonella* possess mutations in genes which encode adenylate cyclase (*cya*) add/or the cyclic AMP receptor protein (*crp*).

10. A vaccine according to claim 8, wherein the *Salmonella* possess a mutation in a *phoP* gene that regulates genes that allow the *Salmonella* to survive in macrophages.

11. A vaccine according to any one of claims 1 to 3 or 8 to 10 for use in a method of immunizing an individual for infections by gram negative bacteria.

12. An isolated avirulent *Salmonella* strain which is able to induce immunity to homologous and heterologous *Salmonella* serotypes and to other gram-negative enteric bacteria wherein the strain possesses a mutation in a gene which encodes adenylate cyclase (*cya*) and/or cyclic AMP receptor protein (*crp*), and also possesses a mutation in a gene encoding an enzyme in lipopolysaccharide synthesis which results in a reversibly rough phenotype.

13. An isolated strain according to claim 12 wherein the *Salmonella* is as defined in claim 2 or 3.

14. An isolated avirulent *Salmonella* strain which is able to induce immunity to homologous and heterologous *Salmonella* serotypes and to other gram-negative enteric bacteria, wherein the strain possesses at least one mutation in a gene involved in the global regulation of pathogenicity of the *Salmonella*, and also possesses a mutation in a gene regulating the synthesis of iron-regulated outer membrane proteins (OMP), such that the mutation leads to constitutive expression of iron-regulated OMP.

15. An isolated strain according to claim 14 wherein the *Salmonella* is as defined in claims 8 to 10.

16. Isolated *Salmonella* according to claim 13 selected from the groups consisting of ATCC No. 68165 and ATCC No. 68167.

## Claims (Claims for the following Contracting State(s): ES, GR)

1. Method for preparing a vaccine for treatment of an individual for infections by gram-negative bacteria comprised of live avirulent *Salmonella* which are able to induce immunity to homologous and heterologous *Salmonella* serotypes and to other gram-negative enteric bacteria, wherein the *Salmonella* possess a mutation in a gene which encodes adenylate cyclase (*cya*) and/or cyclic AMP receptor protein (*crp*), and also possess a mutation in a gene encoding an enzyme in lipopolysaccharide synthesis which results in a reversibly rough phenotype, which method comprises either mutating said *cya* and/or *crp* genes in a strain of *Salmonella* which possesses a mutation in a gene encoding an enzyme in lipopolysaccharide synthesis, or mutating said gene encoding an enzyme in lipopolysaccharide synthesis in a strain of *Salmonella* which possesses a mutation in a gene encoding *cya* or *crp*, growing said cells to obtain an amount of said live cells sufficient to improve the resistance of the individual to infection by the gram-negative enteric bacteria, and mixing the *Salmonella* cells with a pharmaceutically acceptable carrier.

2. A method according to claim 1, wherein the avirulent *Salmonella* obtained possess a mutation in a *galE* gene that encodes UDP-galactose epimerase.

3. A method according to claim 1, wherein the *Salmonella* obtained possess a mutation in a *pmi* gene that encodes phosphomannose isomerase.

4. Use of live avirulent *Salmonella*, which possess at least one mutation in a gene involved in the global regulation of pathogenicity of the *Salmonella* and also possess a mutation in a gene encoding an enzyme in lipopolysaccharide synthesis which results in a reversibly rough phenotype, for the manufacture of a medicament for use in inducing immunity to heterologous *Salmonella* serotypes or to gram-negative bacteria other than *Salmonella*.

5. Use according to claim 4, wherein the *Salmonella* possess a mutation in a *phoP* gene that regulates genes that allow the *Salmonella* to survive in macrophages.

6. Use according to claim 4, wherein the *Salmonella* possess a mutation in a *galE* gene that encodes UDP-galactose epimerase.

7. Use according to claim 4, wherein the *Salmonella* possess a mutation in a *pmi* gene that encodes phosphomannose isomerase.

8. A method for preparing a vaccine for treatment of an individual for infections by gram-negative bacteria comprised of live avirulent *Salmonella* which are able to induce immunity to homologous and heterologous *Salmonella* serotypes and to other gram-negative enteric bacteria, wherein the *Salmonella* possess at least one mutation in a gene involved in the global regulation of pathogenicity of the *Salmonella*, and also possess a mutation in a gene regulating the synthesis of iron-regulated outer membrane proteins (OMP), such that the mutation leads to constitutive expression of iron regulated OMP, which method comprises either mutating a gene involved in the global regulation of pathogenicity in a strain of *Salmonella* which possesses a mutation in a gene regulating the synthesis of OMP, or mutating a gene regulating the synthesis of OMP in a strain of *Salmonella* which possesses a mutation in a gene involved in the global regulation of pathogenicity, growing said cells to obtain an amount of said live cells sufficient to improve the resistance of the individual to infection by the gram negative enteric bacteria, and mixing the *Salmonella* cells with a pharmaceutically acceptable carrier.

9. A method according to claim 8, wherein the avirulent *Salmonella* obtained possess mutations in genes which encode adenylate cyclase (*cya*) and/or the cyclic AMP receptor protein (*crp*).

10. A method according to claim 8, wherein the *Salmonella* obtained possess a mutation in a *phoP* gene that regulates genes that allow the *Salmonella* to survive in macrophages.

11. A method according to any one of claims 1 to 3 or 8 to 10 to obtain a vaccine for use in a method of immunizing an individual for infections by gram negative bacteria.

12. A method for preparing an isolated avirulent *Salmonella* strain which is able to induce immunity to homologous and heterologous *Salmonella* serotypes and to other gram-negative enteric bacteria wherein the strain possesses a mutation in a gene which encodes adenylate cyclase (*cya*) and/or cyclic AMP receptor protein (*crp*), and also possesses a mutation in a gene encoding an enzyme m lipopolysaccharide synthesis which results in a reversibly rough phenotype, which method comprises either mutating said *cya* add/or *crp* genes in a strain of *Salmonella* which possesses a mutation in a gene encoding an enzyme in lipopolysaccharide synthesis, or mutating said gene encoding an enzyme in lipopolysaccharide synthesis in a strain of *Salmonella* which possesses a mutation in a gene encoding *cya* or *crp*.

13. A method according to claim 12 wherein the *Salmonella* obtained is as defined in claim 2 or 3.

14. A method for preparing an isolated avirulent *Salmonella* strain which is able to induce immunity to homologous and heterologous *Salmonella* serotypes and to other gram-negative enteric bacteria, wherein the strain possesses at least one mutation in a gene involved in the global regulation of pathogenicity of the *Salmonella*, and also possesses a mutation in a gene regulating the synthesis of iron-regulated outer membrane proteins (OMP), such that the mutation leads to constitutive expression of iron-regulated OMP, which method comprises either mutating a gene involved in the global regulation of pathogenicity in a strain of *Salmonella* which possesses a mutation in a gene regulating the synthesis of OMP, or mutating a gene regulating the synthesis of OMP in a strain of *Salmonella* which possesses a mutation in a gene involved in the global regulation of pathogenicity.

15. A method according to claim 14 wherein the *Salmonella* obtained is as defined in claims 8 to 10.

16. A method according to claim 13 wherein the isolated *Salmonella* are selected from the groups consisting of ATCC No. 68165 and ATCC No. 68167.

17. A vaccine for treatment of an individual for infections by gram-negative bacteria comprised of live avirulent *Salmonella* which are able to induce immunity to homologous and heterologous *Salmonella* serotypes and to other gram-negative enteric bacteria, wherein the *Salmonella* possess a mutation in a gene which encodes adenylate cyclase (*cya*) and/or cyclic AMP receptor protein (*crp*), and also possess a mutation in a gene encoding an enzyme in lipopolysaccharide synthesis which results in a reversibly rough phenotype, the amount of said live cells being sufficient to improve the resistance of the individual to infection by the gram-negative enteric bacteria, the *Salmonella* cells being present in a pharmaceutically acceptable carrier.

18. A vaccine for treatment of an individual for infections by gram-negative bacteria comprised of live avirulent *Salmonella* which are able to induce immunity to homologous and heterologous *Salmonella* serotypes and to other gram-negative enteric bacteria, wherein the *Salmonella* possess at least one mutation in a gene involved in the global regulation or pathogenicity of the *Salmonella*, and also possess a mutation in a gene regulating the synthesis of iron-regulated outer membrane proteins (OMP), such that the mutation leads to constitutive expression of iron regulated OMP, the amount of said live cells being sufficient to improve the resistance of the individual to infection by the gram negative enteric bacteria, the *Salmonella* cells being present in a pharmaceutically acceptable carrier.

19. An isolated avirulent *Salmonella* strain which is able to induce immunity to homologous and heterologous *Salmonella* serotypes and to other gram-negative enteric bacteria wherein the strain possesses a mutation in a gene which encodes adenylate cyclase (*cya*) and/or cyclic AMP receptor (*crp*), and also possesses a mutation in a gene encoding an enzyme in lipopolysaccharide synthesis which results in a reversibly rough phenotype.

20. An isolated avirulent *Salmonella* strain which is able to induce immunity to homologous and heterologous salmonella serotypes and to other gram-negative enteric bacteria, wherein the strain possesses at least one mutation in a gene involved in the global regulation of pathogenicity of the *Salmonella*, and also possesses a mutation in a gene regulating the synthesis of iron-regulated outer membrane proteins (OMP), such that the mutation leads to constitutive expression of iron-regulated OMP.

## Patentansprüche (Patentansprüche für folgende(n) Vertragsstaat(en): AT, BE, CH, LI, DE, DK, FR, GB, IT, NL, SE)

1. Impfstoff zur Behandlung eines Individuums bei Infektionen durch Gram-negative Bakterien, bestehend aus lebenden avirulenten Salmonella-Zellen, welche die Immunität gegenüber homologen und heterologen Salmonella-Serotypen und gegenüber anderen Gram-negativen Enterobakterien zu induzieren vermögen, wobei die Salmonella-Zellen eine Mutation in einem Gen, das Adenylatcyclase (cya) und/oder cyclisches AMP-Rezeptorprotein (crp) codiert, und ferner eine Mutation in einem Gen, das ein Enzym bei der Lipopolysaccharidsynthese codiert, aufweisen, was zu einem reversibel rauhen Phänotyp führt, wobei die Menge an lebenden Zellen ausreicht, um die Resistenz des Individuums gegen Infektionen durch Gram-negative Enterobakterien zu verbessern und die Salmonella-Zellen in einem pharmazeutisch verträglichen Träger vorliegen.

2. Impfstoff nach Anspruch 1, bei dem die avirulenten Salmonella-Zellen eine Mutation in einem galE-Gen, welches die UDP-Galactose-epimerase codiert, aufweisen.

3. Impfstoff nach Anspruch 1, bei dem die Salmonella-Zellen eine Mutation in einem pmi-Gen, das die Phosphomannose-isomerase codiert, aufweisen.

4. Verwendung von lebenden avirulenten Salmonella-Zellen, die wenigstens eine Mutation in einem Gen, das an der Gesamtsteuerung der Pathogenität von Salmonella teilnimmt, und ferner eine Mutation in einem Gen, das ein Enzym bei der Lipopolysaccharidsynthese codiert, aufweisen, was zu einem reversibel rauhen Phänotyp führt, zur Herstellung eines Medikaments für die Induzierung von Immunität gegenüber heterologen Salmonella-Serotypen oder anderen Gram-negativen Bakterien als Salmonella.

5. Verwendung gemäß Anspruch 4, bei der die Salmonella-Zellen eine Mutation in einem PhoP-Gen aufweisen, das Gene steuert, die es Salmonella ermöglichen, in Macrophagen zu überleben.

6. Verwendung nach Anspruch 4, bei der die Salmonella-Zellen eine Mutation in einem galE-Gen aufweisen, das UDP-Galactose-epimerase codiert.

7. Verwendung nach Anspruch 4, bei der die Salmonella-Zellen eine Mutation in einem pmi-Gen aufweisen, das Phosphomannose-isomerase codiert.

8. Impfstoff zur Behandlung eines Individuums bei Infektionen durch Gram-negative Bakterien, bestehend aus lebenden avirulenten Salmonella-Zellen, welche die Immunität gegenüber homologen und heterologen Salmonella-Serotypen und gegenüber anderen Gram-negativen Enterobakterien zu induzieren vermögen, wobei die Salmonella-Zellen wenigstens eine Mutation in einem Gen, das an der Gesamtsteuerung der Pathogenität von Salmonella teilnimmt, und ferner eine Mutation in einem Gen, das die Synthese der durch Eisen gesteuerten äußeren Membranproteine (OMP) steuert, so daß die Mutation zu einer konstitutiven Expression der durch Eisen gesteuerten OMP führt, aufweisen, wobei die Menge an lebenden Zellen ausreicht, um die Resistenz des Individuums gegen Infektionen durch Gram-negative Enterobakterien zu verbessern und die Salmonella-Zellen in einem pharmazeutisch verträglichen Träger vorliegen.

9. Impfstoff nach Anspruch 8, bei dem die avirulenten Salmonella-Zellen Mutationen in Genen aufweisen, die Adenylatcyclase (cya) und/oder cyclisches AMP-Rezeptorprotein (crp) codieren.

10. Impfstoff nach Anspruch 8, bei dem die Salmonella-Zellen eine Mutation in einem phoP-Gen aufweisen, das die Gene steuert, die Salmonella das Überleben in Macrophagen ermöglichen.

11. Impfstoff nach einem der Ansprüche 1 bis 3 bzw. 8 bis 10 zur Verwendung in einem Verfahren zur Immunisierung eines Individuums bei Infektionen durch Gram-negative Bakterien.

12. Isolierter avirulenter Salmonella-Stamm, welcher die Immunität gegenüber homologen und heterologen Salmonella-Serotypen und gegenüber anderen Gram-negativen Enterobakterien zu induzieren vermag, wobei der Stamm eine Mutation in einem Gen, das Adenylatcyclase (cya) und/oder cyclisches AMP-Rezeptorprotein (crp) codiert, und ferner eine Mutation in einem Gen, das ein Enzym bei der Lipopolysaccharidsynthese codiert, aufweist, was zu einem reversibel rauhen Phänotyp führt.

13. Isolierter Stamm nach Anspruch 12, wobei Salmonella wie in Anspruch 2 oder 3 definiert ist.

14. Isolierter avirulenter Salmonella-Stamm, welcher die Immunität gegenüber homologen und heterologen Salmonella-Serotypen und gegenüber anderen Gram-negativen Enterobakterien zu induzieren vermag, wobei der Stamm wenigstens eine Mutation in einem Gen, das an der Gesamtsteuerung der Pathogenität von Salmonella teilnimmt, und ferner eine Mutation in einem Gen, das die Synthese der durch Eisen gesteuerten äußeren Membranproteine (OMP) steuert, so daß die Mutation zu einer konstitutiven Expression der durch Eisen gesteuerten OMP führt, aufweist.

15. Isolierter Stamm nach Anspruch 14, wobei Salmonella wie in Anspruch 8 bis 10 definiert ist.

16. Isolierte Salmonella-Zellen nach Anspruch 13, ausgewählt aus der Gruppe, bestehend aus ATCC Nr. 68165 und ATCC Nr. 68167.

## Patentansprüche (Patentansprüche für folgende(n) Vertragsstaat(en): ES, GR)

1. Verfahren zur Herstellung eines Impfstoffs zur Behandlung eines Individuums bei Infektionen durch Gram-negative Bakterien, bestehend aus lebenden avirulenten Salmonella-Zellen, welche die Immunität gegenüber homologen und heterologen Salmonella-Serotypen und gegenüber anderen Gram-negativen Enterobakterien zu induzieren vermögen, wobei die Salmonella-Zellen eine Mutation in einem Gen, das Adenylatcyclase (cya) und/oder cyclisches AMP-Rezeptorprotein (crp) codiert, und ferner eine Mutation in einem Gen, das ein Enzym bei der Lipopolysaccharidsynthese codiert, aufweisen, was zu einem reversibel rauhen Phänotyp führt, wobei das Verfahren entweder die Mutation dieser cya- und/oder crp-Gene in einem Stamm von Salmonella, welcher eine Mutation in einem Gen, das ein Enzym bei der Lipopolysaccharidsynthese codiert, oder die Mutation des Gens, das ein Enzym bei der Lipopolysaccharidsynthese codiert, in einem Salmonella-Stamm, der eine Mutation in einem cya oder crp codierenden Gen aufweist, die Züchtung der Zellen zur Erzielung einer Menge an lebenden Zellen die ausreicht, um die Resistenz des Individuums gegen Infektionen durch Gram-negative Enterobakterien zu verbessern und das Mischen der Salmonella-Zellen mit einem pharmazeutisch verträglichen Träger umfaßt.

2. Verfahren nach Anspruch 1, bei dem die erhaltenen avirulenten Salmonella-Zellen eine Mutation in einem galE-Gen aufweisen, das die UDP-Galactose-epimerase codiert.

3. Verfahren nach Anspruch 1, bei dem die erhaltenen Salmonella-Zellen eine Mutation in einem pmi-Gen aufweisen, das die Phosphomannose-isomerase codiert.

4. Verwendung lebender avirulenter Salmonella-Zellen, die wenigstens eine Mutation in einem Gen, das an der Gesamtsteuerung der Pathogenität von Salmonella teilnimmt, und ferner eine Mutation in einem Gen, das ein Enzym bei der Lipopolysaccharidsynthese codiert, aufweisen, was zu einem reversibel rauhen Phänotyp führt, zur Herstellung eines Medikaments für die Induzierung von Immunität gegenüber heterologen Salmonella-Serotypen oder anderen Gram-negativen Bakterien als Salmonella.

5. Verwendung gemäß Anspruch 4, bei der die Salmonella-Zellen eine Mutation in einem phoP-Gen aufweisen, das Gene steuert, die es Salmonella ermöglichen, in Macrophagen zu überleben.

6. Verwendung nach Anspruch 4, bei der die Salmonella-Zellen eine Mutation in einem galE-Gen aufweisen, das UDP-Galactose-epimerase codiert.

7. Verwendung nach Anspruch 4, bei der die Salmonella-Zellen eine Mutation in einem pmi-Gen aufweisen, das die Phosphomannose-isomerase codiert.

8. Verfahren zur Herstellung eines Impfstoffs zur Behandlung eines Individuums bei Infektionen durch Gram-negative Bakterien, bestehend aus lebenden avirulenten Salmonella-Zellen, welche die Immunität gegenüber homologen und heterologen Salmonella-Serotypen und gegenüber anderen Gram-negativen Enterobakterien zu induzieren vermögen, wobei die Salmonella-Zellen wenigstens eine Mutation in einem Gen, das an der Gesamtsteuerung der Pathogenität von Salmonella teilnimmt, und ferner eine Mutation in einem Gen, das die Synthese der durch Eisen gesteuerten äußeren Membranproteine (OMP) steuert, so daß die Mutation zu einer konstitutiven Expression der durch Eisen gesteuerten OMP führt, aufweisen, wobei das Verfahren entweder die Mutation eines Gens, das an der Gesamtsteuerung der Pathogenität teilnimmt, in einem Stamm von Salmonella, der eine Mutation in einem die Synthese von OMP steuernden Gen aufweist, oder die Mutation eines Gens, das die Synthese von OMP steuert, in einem Stamm von Salmonella, der eine Mutation in einem Gen aufweist, das an der Gesamtsteuerung der Pathogenität teilnimmt, die Züchtung der Zellen zur Erzielung einer Menge an lebenden Zellen die ausreicht, um die Resistenz des Individuums gegen Infektionen durch Gram-negative Enterobakterien zu verbessern und das Mischen der Salmonella-Zellen mit einem pharmazeutisch verträglichen Träger umfaßt.

9. Verfahren nach Anspruch 8, bei dem die erhaltenen avirulenten Salmonella-Zellen Mutationen in Genen aufweisen, welche die Adenylat-cyclase (cya) und/oder das cyclische AMP-Rezeptorprotein (crp) codieren.

10. Verfahren nach Anspruch 8, bei dem die erhaltenen Salmonella-Zellen eine Mutation in einem phoP-Gen aufweisen, das die Gene steuert, die Salmonella das Überleben in Macrophagen ermöglichen.

11. Verfahren nach einem der Ansprüche 1 bis 3 bzw. 8 bis 10 zur Herstellung eines Impfstoffs für ein Verfahren zur Immunisierung eines Individuums bei Infektionen durch Gram-negative Bakterien.

12. Verfahren zur Herstellung eines isolierten avirulenten Salmonella-Stammes, welcher die Immunität gegenüber homologen und heterologen Salmonella-Serotypen und gegenüber anderen Gram-negativen Enterobakterien zu induzieren vermag, wobei der Stamm eine Mutation in einem Gen, das Adenylatcyclase (cya) und/oder cyclisches AMP-Rezeptorprotein (crp) codiert, und ferner eine Mutation in einem Gen, das ein Enzym bei der Lipopolysaccharidsynthese codiert, aufweist, was zu einem reversibel rauhen Phänotyp führt, wobei das Verfahren entweder die Mutation dieser cya- und/oder crp-Gene in einem Stamm von Salmonella, welcher eine Mutation in einem Gen, das ein Enzym bei der Lipopolysaccharidsynthese codiert, oder die Mutation des Gens, das ein Enzym bei der Lipopolysaccharidsynthese codiert, in einem Salmonella-Stamm, der eine Mutation in einem cya oder crp-codierenden Gen aufweist, umfaßt.

13. Verfahren nach Anspruch 12, wobei Salmonella wie in Anspruch 2 oder 3 definiert ist.

14. Verfahren zur Herstellung eines isolierten avirulenten Salmonella-Stamms, welcher die Immunität gegenüber homologen und heterologen Salmonella-Serotypen und gegenüber anderen Gram-negativen Enterobakterien zu induzieren vermag, wobei der Stamm wenigstens eine Mutation in einem Gen, das an der Gesamtsteuerung der Pathogenität von Salmonella teilnimmt, und ferner eine Mutation in einem Gen, das die Synthese der durch Eisen gesteuerten äußeren Membranproteine (OMP) steuert, so daß die Mutation zu einer konstitutiven Expression der durch Eisen gesteuerten OMP führt, aufweist, wobei das Verfahren entweder die Mutation eines Gens, das an der Gesamtsteuerung der Pathogenität teilnimmt, in einem Stamm von Salmonella, der eine Mutation in einem die Synthese von OMP steuernden Gen aufweist, oder die Mutation eines Gens, das die Synthese von OMP steuert, in einem Stamm von Salmonella, der eine Mutation in einem Gen aufweist, das an der Gesamtsteuerung der Pathogenität teilnimmt, umfaßt.

15. Verfahren nach Anspruch 14, wobei Salmonella wie in Anspruch 8 bis 10 definiert ist.

16. Verfahren nach Anspruch 13, bei dem die isolierten Salmonella-Zellen nach Anspruch 13 ausgewählt sind aus der Gruppe, bestehend aus ATCC Nr. 68165 und ATCC Nr. 68167.

17. Impfstoff zur Behandlung eines Individuums bei Infektionen durch Gram-negative Bakterien, bestehend aus lebenden avirulenten Salmonella-Zellen, welche die Immunität gegenüber homologen und heterologen Salmonella-Serotypen und gegenüber anderen Gram-negativen Enterobakterien zu induzieren vermögen, wobei die Salmonella-Zellen eine Mutation in einem Gen, das Adenylatcyclase (cya) und/oder cyclisches AMP-Rezeptorprotein (crp) codiert, und ferner eine Mutation in einem Gen, das ein Enzym bei der Lipopolysaccharidsynthese codiert, aufweisen, was zu einem reversibel rauhen Phänotyp führt, wobei die Menge an lebenden Zellen ausreicht, um die Resistenz des Individuums gegen Infektionen durch Gram-negative Enterobakterien zu verbessern und die Salmonella-Zellen in einem pharmazeutisch verträglichen Träger vorliegen.

18. Impfstoff zur Behandlung eines Individuums bei Infektionen durch Gram-negative Bakterien, bestehend aus lebenden avirulenten Salmonella-Zellen, welche die Immunität gegenüber homologen und heterologen Salmonella-Serotypen und gegenüber anderen Gram-negativen Enterobakterien zu induzieren vermögen, wobei die Salmonella-Zellen wenigstens eine Mutation in einem Gen, das an der Gesamtsteuerung der Pathogenität von Salmonella teilnimmt, und ferner eine Mutation in einem Gen, das die Synthese der durch Eisen gesteuerten äußeren Membranproteine (OMP) steuert, so daß die Mutation zu einer konstitutiven Expression der durch Eisen gesteuerten OMP führt, aufweist, wobei die Menge an lebenden Zellen ausreicht, um die Resistenz des Individuums gegen Infektionen durch Gram-negative Enterobakterien zu verbessern und die Salmonella-Zellen in einem pharmazeutisch verträglichen Träger vorliegen.

19. Isolierter avirulenter Salmonella-Stamm, welcher die Immunität gegenüber homologen und heterologen Salmonella-Serotypen und gegenüber anderen Gram-negativen Enterobakterien zu induzieren vermag, wobei der Stamm eine Mutation in einem Gen, das Adenylatcyclase (cya) und/oder cyclisches AMP-Rezeptorprotein (crp) codiert, und ferner eine Mutation in einem Gen, das ein Enzym bei der Lipopolysaccharidsynthese codiert, aufweist, was zu einem reversibel rauhen Phänotyp führt.

20. Isolierter avirulenter Salmonella-Stamm, welcher die Immunität gegenüber homologen und heterologen Salmonella-Serotypen und gegenüber anderen Gram-negativen Enterobakterien zu induzieren vermag, wobei der Stamm wenigstens eine Mutation in einem Gen, das an der Gesamtsteuerung der Pathogenität von Salmonella teilnimmt, und ferner eine Mutation in einem Gen, das die Synthese der durch Eisen gesteuerten äußeren Membranproteine (OMP) steuert, so daß die Mutation zu einer konstitutiven Expression der durch Eisen gesteuerten OMP führt, aufweist.

## Revendications (Revendications pour l'(les) Etat(s) contractant(s) suivant(s): AT, BE, CH, LI, DE, DK, FR, GB, IT, NL, SE)

1. Vaccin pour le traitement d'un individu contre des infections par des bactéries gram-négatives, comprenant des *Salmonella* avirulentes vivantes, capables d'induire une immunité contre des sérotypes de *Salmonella* homologues et hétérologues et contre d'autres bactéries entériques gram-négatives, dans lequel les *Salmonella* possèdent une mutation dans un gène qui code pour l'adénylate cyclase (*cya*) et/ou la protéine réceptrice de l'AMP cyclique (*crp*) et possèdent également une mutation dans un gène codant pour une enzyme de la synthèse des lipopolysaccharides, qui donne un phénotype rugueux (rough) réversible, la quantité desdites cellules vivantes étant suffisante pour améliorer la résistance de l'individu à l'infection par les bactéries entériques gram-négatives, les cellules de *Salmonella* étant présentes dans un véhicule pharmaceutiquement acceptable.

2. Vaccin selon la revendication 1, dans lequel les *Salmonella* avirulentes possèdent une mutation dans un gène *galE* qui code pour l'UDP-galactose épimérase.

3. Vaccin selon la revendication 1, dans lequel les *Salmonella* possèdent une mutation dans un gène *pmi* qui code pour la phosphomannose isomérase.

4. Utilisation de *Salmonella* avirulentes vivantes, qui possèdent au moins une mutation dans un gène impliqué dans la régulation globale du pouvoir pathogène des *Salmonella*, et qui possèdent également une mutation dans un gène codant pour une enzyme de la synthèse des lipopolysaccharides qui donne un phénotype rugueux réversible, pour la fabrication d'un médicament destiné à être utilisé pour induire une immunité contre des sérotypes de *Salmonella* hétérologues ou des bactéries gram-négatives autres que *Salmonella*.

5. Utilisation selon la revendication 4, dans laquelle les *Salmonella* possèdent une mutation dans un gène *phoP* qui régule les gènes permettant aux *Salmonella* de survivre dans les macrophages.

6. Utilisation selon la revendication 4, dans laquelle les *Salmonella* possèdent une mutation dans un gène *galE* qui code pour l'UDP-galactose épimérase.

7. Utilisation selon la revendication 4, dans laquelle les *Salmonella* possèdent une mutation dans un gène *pmi* qui code pour la phosphomannose isomérase.

8. Vaccin pour le traitement d'un individu contre des infections par des bactéries gram-négatives, comprenant des *Salmonella* avirulentes vivantes, qui sont capables d'induire une immunité contre des sérotypes de *Salmonella* homologues et hétérologues et contre d'autres bactéries entériques gram-négatives, dans lequel les *Salmonella* possèdent au moins une mutation dans un gène impliqué dans la régulation globale du pouvoir pathogène des *Salmonella* et possèdent également une mutation dans un gène régulant la synthèse de protéines de la membrane externe (outer membrane protein : OMP) régulées par le fer, de telle sorte que la mutation entraîne l'expression constitutive OMP régulées par le fer, la quantité desdites cellules vivantes étant suffisante pour améliorer la résistance de l'individu à l'infection par les bactéries entériques gram-négatives, les cellules de *Salmonella* étant présentes dans un véhicule pharmaceutiquement acceptable.

9. Vaccin selon la revendication 8, dans lequel les *Salmonella* avirulentes possèdent des mutations dans des gènes qui codent pour l'adénylate cyclase (*cya*) et/ou la protéine réceptrice de l'AMP cyclique (*crp*).

10. Vaccin selon la revendication 8, dans lequel les *Salmonella* possèdent une mutation dans un gène *phoP* qui régule les gènes permettant aux *Salmonella* de survivre dans les macrophages.

11. Vaccin selon l'une quelconque des revendications 1 à 3 ou 8 à 10, destiné à être utilisé dans une méthode d'immunisation d'un individu contre des infections par des bactéries gram-négatives.

12. Souche de *Salmonella* avirulente isolée qui est capable d'induire une immunité contre des sérotypes de *Salmonella* homologues et hétérologues et contre d'autres bactéries entériques gram-négatives, où la souche possède une mutation dans un gêne qui code pour l'adénylate cyclase (*cya*) et/ou la protéine réceptrice de l'AMP cyclique (*crp*) et qui possède également une mutation dans un gène codant pour une enzyme de la synthèse des lipopolysaccharides qui donne un phénotype rugueux réversible.

13. Souche isolée selon la revendication 12, où la *Salmonella* est telle que définie dans la revendication 2 ou la revendication 3.

14. Souche de *Salmonella* avirulente isolée capable d'induire une immunité contre des sérotypes de *Salmonella* homologues et hétérologues et contre d'autres bactéries entériques gram-négatives, la souche possédant au moins une mutation dans un gène impliqué dans la régulation globale du pouvoir pathogène des *Salmonella* et possédant également une mutation dans un gène régulant la synthèse de protéines de la membrane externe (OMP) régulées par le fer, de telle sorte que la mutation entraîne une expression constitutive d'OMP régulées par le fer.

15. Souche isolée selon la revendication 14 où la *Salmonella* est telle que définie dans les revendications 8 à 10.

16. *Salmonella* isolée selon la revendication 13, choisie dans le groupe constitué de ATCC No. 68165 et ATCC No. 68167.

## Revendications (Revendications pour l'(les) Etat(s) contractant(s) suivant(s): ES, GR)

1. Méthode de préparation d'un vaccin pour le traitement d'un individu contre des infections par des bactéries gram-négatives, conmprenant des *Salmonella* avirulentes vivantes, capables d'induire une immunité contre des sérotypes de *Salmonella* homologues et hétérologues et contre d'autres bactéries entériques gram-négatives, dans laquelle les *Salmonella* possèdent une mutation dans un gène qui code pour l'adénylate cyclase (*cya*) et/ou la protéine réceptrice de l'AMP cyclique (*crp*), et possèdent également une mutation dans un gène codant pour une enzyme de la synthèse des lipopolysaccharides, qui donne un phénotype rugueux (rough) réversible, méthode qui comprend les étapes consistant soit à effectuer une mutation dans lesdits gènes *cya* et/ou *crp* dans une souche de *Salmonella* qui possède une mutation dans un gène codant pour une enzyme de la synthèse des lipopolysaccharides, soit à effectuer une mutation dudit gène codant pour une enzyme de la synthèse des lipopolysaccharides dans une souche de *Salmonella* qui possède une mutation dans un gène codant pour *cya* ou *crp*, à cultiver lesdites cellules pour obtenir une quantité desdites cellules vivantes suffisante pour améliorer la résistance de l'individu à l'infection par les bactéries entériques gram-négatives, et à mélanger les cellules de *Salmonella* avec un véhicule pharmaceutiquement acceptable.

2. Méthode selon la revendication 1, dans laquelle les *Salmonella* avirulentes obtenues possèdent une mutation dans un gène *galE* qui code pour l'UDP-galactose épimérase.

3. Méthode selon la revendication 1, dans laquelle les *Salmonella* obtenues possèdent une mutation dans un gène *pmi* qui code pour la phosphomannose isomérase.

4. Utilisation de *Salmonella* avirulentes vivantes, qui possèdent au moins une mutation dans un gène impliqué dans la régulation globale du pouvoir pathogène des *Salmonella* et qui possèdent également une mutation dans un gène codant pour une enzyme de la synthèse des lipopolysaccharides qui donne un phénotype rugueux réversible, pour la fabrication d'un médicament destiné à être utilisé pour induire une immunité contre des sérotypes de *Salmonella* hétérologues ou des bactéries gram-négatives autres que *Salmonella*.

5. Utilisation selon la revendication 4, dans laquelle les *Salmonella* possèdent une mutation dans un gène *phoP* qui régule les gènes permettant aux *Salmonella* de survivre dans les macrophages.

6. Utilisation selon la revendication 4, dans laquelle les *Salmonella* possèdent une mutation dans un gène *galE* qui code pour l'UDP-galactose épimérase.

7. Utilisation selon la revendication 4, dans laquelle les *Salmonella* possèdent une mutation dans un gène *pmi* qui code pour la phosphomannose isomérase.

8. Méthode de préparation d'un vaccin pour le traitement d'un individu contre des infections par des bactéries gram-négatives comprenant des *Salmonella* avirulentes vivantes, qui sont capables d'induire une immunité contre des sérotypes de *Salmonella* homologues et hétérologues et contre d'autres bactéries entériques gram-négatives, dans laquelle les *Salmonella* possèdent au moins une mutation dans un gène impliqué dans la régulation globale du pouvoir pathogène des *Salmonella* et possèdent également une mutation dans un gène régulant la synthèse de protéines de la membrane externe (outer membrane protein : OMP) régulées par le fer, de telle sorte que la mutation entraîne l'expression constitutive d'OMP régulées par le fer, méthode qui comprend les étapes consistant soit à effectuer une mutation dans un gène impliqué dans la régulation globale du pouvoir pathogène dans une souche de *Salmonella* qui possède une mutation dans un gène régulant la synthèse d'OMP, soit à effectuer une mutation dans un gène régulant la synthèse d'OMP dans une souche de *Salmonella* qui possède une mutation dans un gène impliqué dans la régulation globale du pouvoir pathogène, à cultiver lesdites cellules pour obtenir une quantité. desdites cellules vivantes suffisante pour améliorer la résistance de l'individu à l'infection par les bactéries entériques gram-négatives, et à mélanger les cellules de *Salmonella* avec un véhicule pharmaceutiquement acceptable.

9. Méthode selon la revendication 8, dans laquelle les *Salmonella* avirulentes obtenues possèdent des mutations dans des gènes qui codent pour l'adénylate cyclase (*cya*) et/ou la protéine réceptrice de l'AMP cyclique (*crp*).

10. Méthode selon la revendication 8, dans laquelle les *Salmonella* obtenues possèdent une mutation dans un gène *phoP* qui régule les gènes permettant aux *Salmonella* de survivre dans les macrophages.

11. Méthode selon l'une quelconque des revendications 1 à 3 ou 8 à 10, pour obtenir un vaccin destiné à être utilisé dans une méthode d'immunisation d'un individu contre des infections par des bactéries gram-négatives.

12. Méthode de préparation d'une souche de *Salmonella* avirulente isolée qui est capable d'induire une immunité contre des sérotypes de *Salmonella* homologues et hétérologues et contre d'autres bactéries entériques gram-négatives, dans laquelle la souche possède une mutation dans un gène qui code pour l'adénylate cyclase (*cya*) et/ou la protéine réceptrice de l'AMP cyclique (*crp*) et qui possède également une mutation dans un gène codant pour une enzyme de la synthèse des lipopolysaccharides qui donne un phénotype rugueux réversible, méthode qui comprend les étapes consistant soit à effectuer une mutation dans lesdits gènes *cya* et/ou *crp* dans une souche de *Salmonella* qui possède une mutation dans un gène codant pour une enzyme de la synthèse des lipopolysaccharides, soit à effectuer une mutation dudit gène codant pour une enzyme de la synthèse des lipopolysaccharides dans une souche de *Salmonella* qui possède une mutation dans un gène codant pour *cya* ou *crp*.

13. Méthode selon la revendication 12, dans laquelle la *Salmonella* obtenue est telle que définie dans la revendication 2 ou la revendication 3.

14. Méthode de préparation d'une souche de *Salmonella* avirulente isolée capable d'induire une immunité contre des sérotypes de *Salmonella* homologues et hétérologues et contre d'autres bactéries entériques gram-négatives, dans laquelle la souche possède au moins une mutation dans un gène impliqué dans la régulation globale du pouvoir pathogène des *Salmonella* et posséde également une mutation dans un gène régulant la synthèse de protéines de la membrane externe (OMP) régulées par le fer, de telle sorte que la mutation entraîne une expression constitutive d'OMP régulées par le fer, méthode qui comprend les étapes consistant soit à effectuer une mutation dans un gène impliqué dans la régulation globale du pouvoir pathogène dans une souche de *Salmonella* qui possède une mutation dans un gène régulant la synthèse d'OMP, soit à effectuer une mutation dans un gène régulant la synthèse d'OMP dans une souche de *Salmonella* qui possède une mutation dans un gène impliqué dans la régulation global du pouvoir pathogène.

15. Méthode selon la revendication 14, dans laquelle la *Salmonella* obtenue est telle que définie dans les revendications 8 à 10.

16. Méthode selon la revendication 13, dans laquelle les *Salmonella* isolées sont choisies dans le groupe constitué de ATCC No. 68165 et ATCC No. 68167.

17. Vaccin pour le traitement d'un individu contre des infections par des bactéries gram-négatives comprenant des *Salmonella* avirulentes vivantes, capables d'induire une immunité contre des sérotypes de *Salmonella* homologues et hétérologues et contre d'autres bactéries entériques gram-négatives, dans lequel les *Salmonella* possèdent une mutation dans un gène qui code pour l'adénylate cyclase (*cya*) et/ou la protéine réceptrice de l'AMP cyclique (*crp*) et possèdent également une mutation dans un gène codant pour une enzyme de la synthèse des lipopolysaccharides, qui donne un phénotype rugueux réversible, la quantité desdites cellules vivantes étant suffisante pour améliorer la résistance de l'individu à l'infection par les bactéries entériques gram-négatives, les cellules de *Salmonella* étant présentes dans un véhicule pharmaceutiquement acceptable.

18. Vaccin pour le traitement d'un individu contre des infections par des bactéries gram-négatives comprenant des *Salmonella* avirulentes vivantes, qui sont capables d'induire une immunité contre des sérotypes de *Salmonella* homologues et hétéroloques et contre d'autres bactéries entériques gram-négatives, dans lequel les *Salmonella* possèdent au moins une mutation dans un gène impliqué dans la régulation globale du pouvoir pathogène des *Salmonella* et possèdent également une mutation dans un gène régulant la synthèse de protéines de la membrane externe (OMP) régulées par le fer, de telle sorte que la mutation entraîne l'expression constitutive d'OMP régulées par le fer, la quantité desdites cellules vivantes étant suffisante pour améliorer la résistance de l'individu à l'infection par les bactéries entériques gram-négatives, les cellules de *Salmonella* étant présentes dans un véhicule pharmaceutiquement acceptable.

19. Souche de *Salmonella* avirulente isolée qui est capable d'induire une immunité contre des sérotypes de *Salmonella* homologues et hétérologues et contre d'autres bactéries entériques gram-négatives, où la souche possède une mutation dans un gène qui code pour l'adénylate cyclase (*cya*) et/ou le récepteur de l'AMP cyclique (*crp*) et qui possède également une mutation dans un gène codant pour une enzyme de la synthèse des lipopolysaccharides qui donne un phénotype rugueux réversible.

20. Souche de *Salmonella* avirulente isolée qui est capable d'induire une immunité contre des sérotypes de *Salmonella* homologues et hétérologues et contre d'autres bactéries entériques gram-négatives, où souche posséde au moins une mutation dans un gène impliqué dans la régulation globale du pouvoir pathogène des *Salmonella* et posséde également une mutation dans un gène régulant la synthèse de protéines de la membrane externe (OMP) régulées par le fer, de telle sorte que la mutation entraîne une expression constitutive d'OMP régulées par le fer.
